(19) Europäisches Patentamt
European Patent Office
Office européen des brevets

(11) **EP 3 199 108 A1**

(12) **EUROPEAN PATENT APPLICATION**

(43) Date of publication:
**02.08.2017 Bulletin 2017/31**

(51) Int Cl.:
**A61B 8/08** (2006.01)

(21) Application number: **16187183.5**

(22) Date of filing: **05.09.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **01.02.2016 KR 20160012456**

(71) Applicant: **Samsung Medison Co., Ltd.
Hongcheon-gun, Gangwon-do, 25108 (KR)**

(72) Inventors:
• **PARK, Jin-ki
Gangwon-do (KR)**
• **PARK, Sung-wook
Gangwon-do (KR)**
• **LEE, Jin-yong
Gangwon-do (KR)**

(74) Representative: **D'Halleweyn, Nele Veerle Trees Gertrudis et al
Arnold & Siedsma
Bezuidenhoutseweg 57
2594 AC The Hague (NL)**

(54) **METHOD AND APPARATUS FOR DISPLAYING ULTRASOUND IMAGE**

(57) A method and an ultrasound imaging apparatus are disclosed, wherein three-dimensional (3D) ultrasound volume data of an object (10) comprising a blood vessel (200) is obtained, and a degree of stenosis is analysed for a plurality of cross-sections. A stenosis cross-section (P210) with a highest degree of stenosis is determined, and a first cross-section (P230) and a second cross-section (P240) that are spaced apart from each other in different directions from the stenosis cross-section are determined. At least two cross-sections of the stenosis cross-section, the first cross-section, the second cross-section, and at least two stenosis index values showing a degree of stenosis of the blood vessel are displayed on a display unit (130) of the ultrasound imaging apparatus, thereby improving diagnostic usability for a user.

FIG. 1

**Description**

CROSS-REFERENCE TO RELATED APPLICATION

This application claims the benefit of Korean Patent Application No. 10-2016-0012456, filed on February 1, 2016, in the Korean Intellectual Property Office, the disclosure of which is incorporated herein in its entirety by reference.

BACKGROUND

1. Field

The present disclosure relates to methods and apparatuses for displaying ultrasound images, and more particularly, to methods and apparatuses for determining a plurality of blood vessel cross-sections related to measurement of a degree of stenosis of the blood vessel from three dimensional (3D) ultrasound volume data and displaying a stenosis index value representing a degree of stenosis of a plurality of blood vessel cross-sections of and of the blood vessel.

2. Description of the Related Art

Due to its non-invasive and non-destructive nature, an ultrasound system has become widely used in many medical fields that require information about the inside of an object. The ultrasound system also plays a critical role in medical diagnosis since it can provide high-resolution images of an inner area of an object to a medical practitioner without the need for performing a surgical procedure to directly incise the object for observation.

In order to measure a degree of stenosis of a blood vessel by using an ultrasound system, an ultrasound probe may be moved over a portion of a patient's body including the blood vessel to find plural cross-sections of the blood vessel, followed by measurement of a diameter or an area of a blood vessel cross-section. As an index for measuring a degree of stenosis by using a diameter of a blood vessel cross-section, North American Symptomatic Carotid Endarterectomy Trial (NASCET), European Carotid Surgery Trial (ECST), or a common carotid method (CC) may be used.

SUMMARY

Provided are methods and apparatuses for displaying ultrasound images of a plurality of blood vessel cross-sections, which are related to measurement of a degree of stenosis of the blood vessel and are obtained from 3D ultrasound volume data, and a stenosis index value calculated from the plurality of cross-sections of the blood vessel, enabling a user to easily recognize the degree of stenosis of the blood vessel, thereby improving diagnostic usability for a user.

Additional aspects will be set forth in part in the description which follows and, in part, will be apparent from the description, or may be learned by practice of the presented embodiments.

According to an aspect of an embodiment, a method, performed by an ultrasound imaging apparatus, of displaying an ultrasound image with respect to an object including a blood vessel, includes obtaining three-dimensional (3D) ultrasound volume data with respect to the object; analyzing a degree of stenosis for a plurality of cross-sections of the blood vessel from the 3D ultrasound volume data and determining a stenosis cross-section with a highest degree of stenosis; determining a first cross-section and a second cross-section that are spaced apart from each other in different directions from the stenosis cross-section from among the plurality of cross-sections of the blood vessel; and displaying at least two cross-sections of the stenosis cross-section, the first cross-section, the second cross-section, and at least two stenosis index values showing a degree of stenosis of the blood vessel on a display unit of the ultrasound imaging apparatus.

The displaying of at least two stenosis index values may include displaying at least two index values from among a first index value representing a ratio of a diameter of the first cross-section to a difference between the diameter of the first cross-section and a diameter of the stenosis cross-section, a second index value representing a ratio of an entire blood vessel diameter defined by a blood vessel wall of the stenosis cross-section to a difference between the entire blood vessel diameter and the diameter of the stenosis cross-section, a third index value representing a ratio of a diameter of the second cross-section to a difference between the diameter of the second cross-section and the diameter of the stenosis cross-section, and a fourth index value representing a ratio of a cross-sectional area of the entire blood vessel of the stenosis cross-section to an area of the stenosis cross-section.

The blood vessel may be a carotid artery, the first cross-section may be a cross-section of an internal carotid artery (ICA), and the second cross-section may be a cross-section of a common carotid artery (CCA).

The ultrasound imaging method may further include rendering the 3D ultrasound volume data to generate a 3D ultrasound image of the object, wherein the displaying of the at least two cross-sections and the at least two stenosis index values may include displaying the at least two cross-sections, the at least two stenosis index values, and the 3D ultrasound image on the display unit.

The displaying of the at least two cross-sections and the at least two stenosis index values may include displaying a user interface that displays at least one of different colors, graphs, and figures based on sizes of the at least two stenosis index values, along with the at least two stenosis index values.

The obtaining of the 3D ultrasound volume data with respect to the object may include transmitting ultrasound signals to the object; receiving ultrasound echo signals reflected by the object; and obtaining the 3D ultrasound volume data from the received ultrasound echo signals.

The determining of the stenosis cross-section may include obtaining the plurality of cross-sections of the blood vessel by segmenting the 3D ultrasound volume data at predetermined intervals; calculating, with respect to each of the plurality of cross-sections of the blood vessel, a first area value regarding a cross-section defined by a blood vessel wall of the blood vessel and a second area value regarding a cross-section through which blood flows in the blood vessel; calculating, with respect to each of the plurality of cross-sections of the blood vessel, a ratio of the first area value to the second area value; and determining a cross-section having a smallest calculated ratio as a stenosis cross-section from among the plurality of cross-sections of the blood vessel.

The calculating of the second area value may include obtaining a brightness value for each of the plurality of cross-sections of the blood vessel; determining an area of a region whose obtained brightness value may be less than a predetermined brightness value as a second area; and calculating an area value of the second area.

The first cross-section may be spaced apart by a predetermined first interval in a first direction from the stenosis cross-section and the second cross-section may be spaced apart by the first interval in a second direction that may be opposite to the first direction from the stenosis cross-section.

The determining of the first cross-section and the second cross-section may include receiving a user input for setting a second interval that may be spaced apart from the stenosis cross-section, wherein the first cross-section may be spaced apart by the second interval in a first direction from the stenosis cross-section and the second cross-section may be spaced apart by the second interval in a second direction that may be opposite to the first direction from the stenosis cross-section.

The determining of the first cross-section and the second cross-section may include detecting from the 3D ultrasound volume data a branch point where the blood vessel diverges into two; and determining a cross-section spaced apart by a third interval in a first direction from the branch point as the first cross-section and determining a cross-section spaced apart by the third interval in a second direction that may be opposite to the first direction from the branch point as the second cross-section.

According to another aspect of an embodiment, an ultrasound imaging apparatus displaying an ultrasound image with respect to an object including a blood vessel includes an ultrasound data acquisition unit configured to obtain 3D ultrasound volume data with respect to the object; a processor configured to analyze a degree of stenosis for a plurality of cross-sections of the blood vessel from the 3D ultrasound volume data, determine a stenosis cross-section with a highest degree of stenosis and determine a first cross-section and a second cross-section that are spaced apart from each other in different directions from the stenosis cross-section from among the plurality of cross-sections of the blood vessel; and a display unit configured to display at least two cross-sections of the stenosis cross-section, the first cross-section, the second cross-section, and at least two stenosis index values showing a degree of stenosis of the blood vessel.

The processor may further be configured to calculate at least two index values from among a first index value representing a ratio of a diameter of the first cross-section to a difference between the diameter of the first cross-section and a diameter of the stenosis cross-section, a second index value representing a ratio of an entire blood vessel diameter defined by a blood vessel wall of the stenosis cross-section to a difference between the entire blood vessel diameter and the diameter of the stenosis cross-section, a third index value representing a ratio of a diameter of the second cross-section to a difference between the diameter of the second cross-section and the diameter of the stenosis cross-section, and a fourth index value representing a ratio of a cross-sectional area of the entire blood vessel of the stenosis cross-section to an area of the stenosis cross-section, and the display unit may further be configured to display at least two index values from among the first index value, the second index value, the third index value, and the fourth index value.

The blood vessel may be a carotid artery, the first cross-section may be a cross-section of an ICA, and the second cross-section may be a cross-section of a CCA.

The processor may further be configured to render the 3D ultrasound volume data to generate a 3D ultrasound image of the object; and the display unit may further be configured to display the at least two cross-sections, the at least two stenosis index values, and the 3D ultrasound image on the display unit.

The display unit may further be configured to display a user interface that displays at least one of different colors, graphs, and figures based on sizes of the at least two stenosis index values, along with the at least two stenosis index values.

The ultrasound data acquisition unit may further be configured to transmit ultrasound signals to the object, receive ultrasound echo signals reflected by the object, and obtain the 3D ultrasound volume data from the received ultrasound echo signals.

The processor may further be configured to obtain the plurality of cross-sections of the blood vessel by segmenting the 3D ultrasound volume data at predetermined intervals, calculate, with respect to each of the plurality of cross-sections of the blood vessel, a first area value regarding a cross-section defined by a blood vessel wall of the blood vessel and a second area value regarding a cross-section through which blood flows in the blood vessel, calculate, with respect to each of the plurality of cross-sections of the blood vessel, a ratio of the first area value to the second area value, and determine a cross-section having a smallest calculated ratio as a stenosis cross-section from among the plurality of cross-sections of the blood vessel.

The processor may further be configured to obtain a brightness value for each of the plurality of cross-sections of the blood vessel, determine an area of a region whose obtained brightness value may be less than a predetermined brightness value as a second area, and calculate an area value of the second area.

The first cross-section may be spaced apart by a predetermined first interval in a first direction from the stenosis cross-section and the second cross-section may be spaced apart by the first interval in a second direction that may be opposite to the first direction from the stenosis cross-section.

The ultrasound imaging apparatus may further include a user input unit configured to receive a user input for setting a second interval that may be spaced apart from the stenosis cross-section, wherein the processor may further be configured to determine the first cross-section spaced apart by the second interval in a first direction from the stenosis cross-section and the second cross-section spaced apart by the second interval in a second direction that may be opposite to the first direction from the stenosis cross-section.

The processor may further be configured to detect from the 3D ultrasound volume data a branch point where the blood vessel diverges into two, determines a cross-section spaced apart by a third interval in a first direction from the branch point as the first cross-section, and determine a cross-section spaced apart by the third interval in a second direction that may be opposite to the first direction from the branch point as the second cross-section.

A non-transitory computer-readable recording medium may have recorded thereon a program for executing the method on a computer.

BRIEF DESCRIPTION OF THE DRAWINGS

These and/or other aspects will become apparent and more readily appreciated from the following description of the embodiments, taken in conjunction with the accompanying drawings in which:

FIG. 1 is a conceptual view for explaining a method, performed by an ultrasound imaging apparatus, of obtaining an ultrasound image with respect to a blood vessel and displaying a plurality of cross-sections of the blood vessel for measurement of a degree of stenosis of the blood vessel and a plurality of stenosis index values, according to an embodiment;
FIG. 2 is a block diagram showing a configuration of an ultrasound imaging apparatus according to an embodiment;

FIG. 3 is a flowchart of a method of displaying a plurality of blood vessel cross-sections related to measurement of a degree of stenosis of the blood vessel and stenosis index values by an ultrasound imaging apparatus according to an embodiment;
FIG. 4A is a diagram for explaining a structure of a blood vessel that is stenosed;
FIG. 4B is a diagram for explaining a method, performed by an ultrasound imaging apparatus, of obtaining a plurality of blood vessel cross-sections from 3D ultrasound volume data, according to an embodiment;
FIG. 4C is a diagram for explaining a method, performed by an ultrasound imaging apparatus, of calculating a degree of stenosis for a plurality of blood vessel cross-sections, according to an embodiment;
FIG. 5 is a flowchart of a method, performed by an ultrasound imaging apparatus, of determining a stenosis cross-section from among a plurality of blood vessel cross-sections, according to an embodiment;
FIG. 6 is a diagram for explaining a method, performed by an ultrasound imaging apparatus, of determining a plurality of blood vessel cross-sections for measurement of a degree of stenosis of the blood vessel, according to an embodiment;
FIG. 7 is a block diagram showing a configuration of an ultrasound imaging apparatus according to an embodiment;
FIG. 8 is a flowchart of a method, performed by an ultrasound imaging apparatus, of determining a plurality of blood vessel cross-sections for measurement of a degree of stenosis of the blood vessel, according to an embodiment;
FIG. 9 is a diagram for explaining a method, performed by an ultrasound imaging apparatus, of determining a plurality of blood vessel cross-sections for measurement of a degree of stenosis of the blood vessel according to an embodiment;
FIG. 10 is a flowchart of a method, performed by an ultrasound imaging apparatus, of determining a plurality of blood vessel cross-sections for measurement of a degree of stenosis of the blood vessel, according to an embodiment;
FIG. 11A is a diagram for explaining a method, performed by an ultrasound imaging apparatus, of calculating a stenosis index value representing a degree of stenosis of a blood vessel, according to an embodiment;
FIG. 11B is a cross-sectional view of a blood vessel for explaining a method, performed by an ultrasound imaging apparatus, of calculating a degree of stenosis of the blood vessel, according to an embodiment;
FIGS. 12A to 12D are diagrams for explaining a method, performed by an ultrasound imaging apparatus, of displaying a plurality of blood vessel cross-sections for measurement of a degree of stenosis of the blood vessel and at least two stenosis index values representing the degree of stenosis of the blood vessel, according to an embodiment;
FIG. 13 is a block diagram showing a configuration of an ultrasound diagnosis apparatus according to an embodiment; and
FIG. 14 is a block diagram showing a configuration of a wireless probe according to an embodiment.

DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects thereof. Expressions such as "at least one of," when preceding a list of elements, modify the entire list of elements and do not modify the individual elements of the list.

The attached drawings for illustrating exemplary embodiments of the present disclosure are referred to in order to gain a sufficient understanding of the present disclosure, the merits thereof, and the objectives accomplished by the implementation of the present disclosure. In this regard, the present embodiments may have different forms and should not be construed as being limited to the descriptions set forth herein. Rather, these embodiments are provided so that this disclosure will be thorough and complete and will fully convey the concept of the present embodiments to one of ordinary skill in the art, and the spirit and scope of the present invention will only be defined by the appended claims.

Hereinafter, the terms used in the specification will be briefly described, and then the present disclosure will be described in detail.

The terms used in this specification are those general terms currently widely used in the art in consideration of functions regarding the present disclosure, but the terms may vary according to the intention of those of ordinary skill in the art, precedents, or new technology in the art. Also, some terms may be arbitrarily selected by the applicant, and in this case, the meaning of the selected terms will be described in detail in the detailed description of the present specification. Thus, the terms used in the specification should be understood not as simple names but based on the meaning of the terms and the overall description of the invention.

When a part "includes" or "comprises" an element, unless there is a particular description contrary thereto, the part can further include other elements, not excluding the other elements. Also, the term "unit" in the embodiments of the present invention means a software component or hardware component such as a field-programmable gate array (FPGA) or an application-specific integrated circuit (ASIC), and performs a specific function. However, the term "unit" is not limited to software or hardware. The "unit" may be formed so as to be in an addressable storage medium, or may be formed so as to operate one or more processors. Thus, for example, the term "unit" may refer to components such as software components, object-oriented software components, class components, and task components, and may include processes, functions, attributes, procedures, subroutines, segments of program code, drivers, firmware, micro codes, circuits, data, a database, data structures, tables, arrays, or variables. A function provided by the components and "units" may be associated with the smaller number of components and "units", or may be divided into additional components and "units".

Throughout the specification, an "ultrasound image" refers to an image of an object, which is obtained using ultrasound waves. Furthermore, an "object" may be a human, an animal, or a part of a human or animal. For example, the object may be an organ (e.g., the liver, the heart, the womb, the brain, a breast, or the abdomen), a blood vessel, or a combination thereof. Also, the object may be a phantom. The phantom means a material having a density, an effective atomic number, and a volume that are approximately the same as those of an organism. For example, the phantom may be a spherical phantom having properties similar to a human body.

Throughout the specification, a "user" may be, but is not limited to, a medical expert, for example, a medical doctor, a nurse, a medical laboratory technologist, or a medical imaging expert, or a technician who repairs medical apparatuses.

Furthermore, in the present specification, the terms "first", "second", "1-1", etc. are only used to distinguish one component, element, image, pixel, or patch from another component, element, object, image, pixel, or patch. Thus, these terms are not limited to representing the order or priority among elements or components.

Reference will now be made in detail to embodiments, examples of which are illustrated in the accompanying drawings. In the following description, well-known functions or constructions are not described in detail so as not to obscure the embodiments with unnecessary detail.

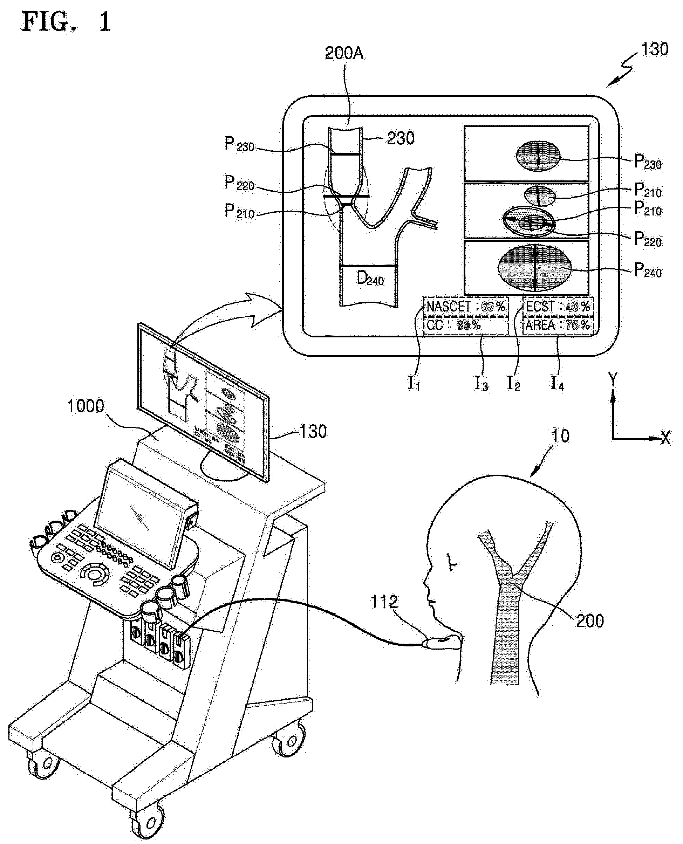

FIG. 1 is a conceptual view for explaining a method, performed by an ultrasound imaging apparatus 100, of obtaining an ultrasound image with respect to a blood vessel 200 and displaying a plurality of blood vessel cross-sections $P_{210}$, $P_{220}$ $P_{230}$ and $P_{240}$ for measurement of a degree of stenosis of the blood vessel 200 and a plurality of stenosis index values $I_1$. $I_2$, $I_3$, and $I_4$, according to an embodiment.

Referring to FIG. 1, the ultrasound imaging apparatus 100 may display a three dimensional (3D) ultrasound image 200A of an object 10 including the blood vessel 200, the plurality of blood vessel cross-sections $P_{21o}$, $P_{220}$, $P_{230}$, and $P_{240}$, and the plurality of stenosis index values $I_1$, $I_2$, $I_3$, and $I_4$ on a display unit 130. However, embodiments are not limited to the illustration of FIG. 1; and the ultrasound imaging apparatus 100 may display at least two stenosis index values from among the plurality of stenosis index values $I_1$, $I_2$, $I_3$, and $I_4$.

In an embodiment, the blood vessel 200 may be a carotid artery located in the neck of the object 10, i.e., a patient. A user using the ultrasound imaging apparatus 100 may scan a neck part of the object 10 by using an ultrasound probe 112 to obtain 3D ultrasound volume data with respect to the blood vessel 200. In particular, the ultrasound probe 112 may transmit ultrasound signals to the object 10, the ultrasound imaging apparatus 100 may receive ultrasound echo signals reflected by the object 10, and the 3D ultrasound image 200A may be generated by performing a scan conversion on the received ultrasound echo signals.

The ultrasound imaging apparatus 100 may obtain a plurality of cross-sections with respect to the blood vessel 200 from 3D ultrasound volume data of the object 10 and determine stenosis cross-sections $P_{210}$ and $P_{220}$ with a highest degree of stenosis by analyzing degrees of stenosis from among a plurality of cross-sections. In an embodiment, the ultrasound imaging apparatus 100 may receive a user input for selecting the stenosis cross-sections $P_{210}$ and $P_{220}$ in the blood vessel 200 and determine the stenosis cross-sections $P_{210}$ and $P_{220}$ based on the user input.

The ultrasound imaging apparatus 100 may determine a first cross-section $P_{230}$ and a second cross-section $P_{240}$ that may be each spaced apart in different directions from the determined stenosis cross-sections $P_{210}$ and $P_{220}$. In an embodiment, the first cross-section $P_{230}$ may be spaced apart by a predetermined interval in a first direction Y from the stenosis cross-sections $P_{210}$ and $P_{220}$ and the second cross-section $P_{240}$ may be spaced apart by a predetermined interval in a second direction that is opposite to the first direction Y from the stenosis cross-sections $P_{210}$ and $P_{220}$.

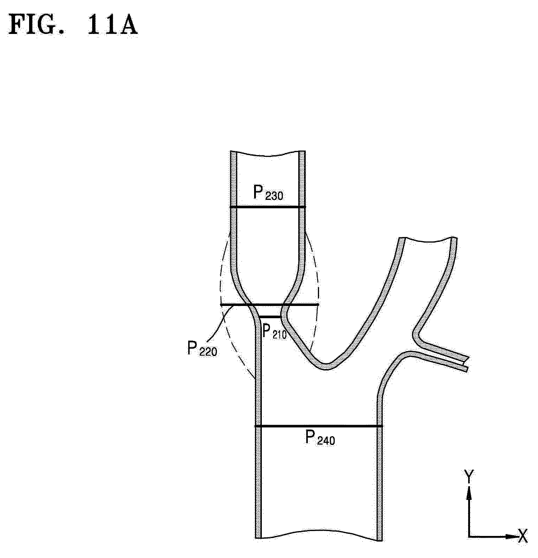

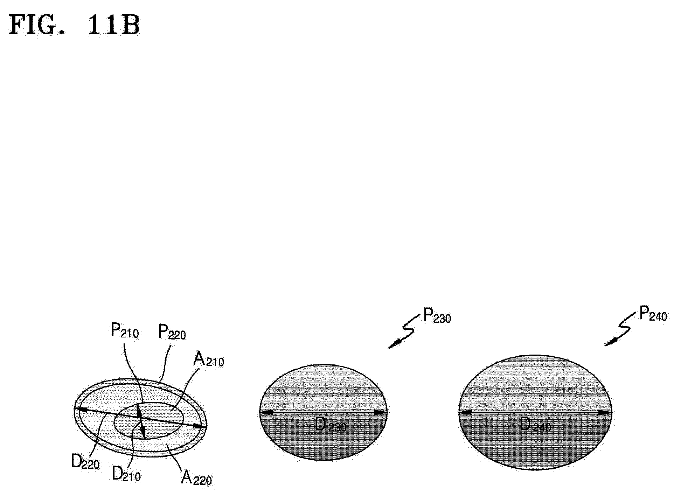

The ultrasound imaging apparatus 100 may calculate the plurality of stenosis index values $I_1$, $I_2$, $I_3$, and $I_4$ representing a degree of stenosis of the blood vessel 200 based on a diameter or an area value of the stenosis cross-sections $P_{210}$ and $P_{220}$, the first cross-section $P_{230}$, and the second cross-section $P_{240}$. In an embodiment, the plurality of stenosis index values $I_1$, $I_2$, $I_3$, and $I_4$ may include at least one of North American Symptomatic Carotid Endarterectomy Trial (NASCET), European Carotid Surgery Trial (ECST), and a common carotid method (CC). The plurality of stenosis index values $I_1$, $I_2$, $I_3$, and $I_4$ are described below in relation to FIGS. 11A and 11B.

The ultrasound imaging apparatus 100 may display at least two cross-sections of the calculated plurality of stenosis index values $I_1$, $I_2$, $I_3$, and $I_4$, the stenosis cross-sections $P_{210}$ and $P_{220}$, the first cross-section $P_{230}$, and the second cross-section $P_{240}$ on the display unit 130. In an embodiment, the ultrasound imaging apparatus 100 may render 3D ultrasound volume data to generate the 3D ultrasound image 200A with respect to the blood vessel 200 and display the generated 3D ultrasound image 200A on the display unit 130.

In general, in the case of a two-dimensional (2D) ultrasound imaging apparatus using a one-dimensional (1D) ultrasound probe, in order to obtain ultrasound data for a plurality of cross-sections of the blood vessel 200, the object 10 including the blood vessel 200 may be scanned several times. Even in the case of a 3D ultrasound imaging apparatus, in order to obtain ultrasound data for a plurality of cross-sections of the blood vessel 200, it is inconvenient to manually adjust the position of a cross-section.

The ultrasound imaging apparatus 100 according to an embodiment may obtain ultrasound data for the stenosis cross-sections $P_{210}$ and $P_{220}$, with a highest degree of stenosis in the blood vessel 200 and automatically obtain the first cross-section $P_{230}$ and the second cross-section $P_{240}$ with respect to the stenosis cross-sections $P_{210}$ and $P_{220}$. In addition, the ultrasound imaging apparatus 100 according to an embodiment may display the plurality of stenosis index values $I_1$, $I_2$, $I_3$, and $I_4$ calculated based on a diameter or an area value of the stenosis cross-sections $P_{210}$ and $P_{220}$, the first cross-section $P_{230}$, and the second cross-section $P_{240}$ together on the display unit 130, enabling a user to determine a degree of stenosis of the blood vessel 200 and improving diagnostic usability for the user.

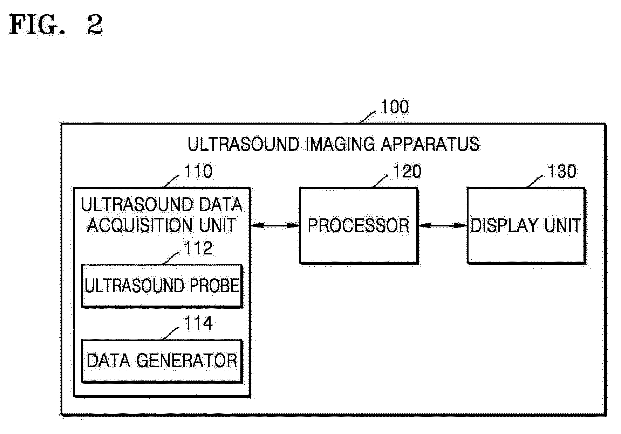

FIG. 2 is a block diagram showing a configuration of an ultrasound imaging apparatus 100 according to an embodiment. In the ultrasound imaging apparatus 100 of FIG. 2, only elements that are related to the present embodiment are illustrated. Therefore, it will be appreciated that other generic components may further be included, in addition to the elements shown in FIG. 2, by those of ordinary skill in the art.

Referring to FIG. 2, the ultrasound imaging apparatus 100 may include an ultrasound data acquisition unit 110, a processor 120, and the display unit 130. In an embodiment, the ultrasound imaging apparatus 100 may be a cart type apparatus or a portable type apparatus. Examples of portable the ultrasound imaging apparatus 100 may include, but are not limited to, a picture archiving and communication system (PACS) viewer, a smartphone, a laptop computer, a personal digital assistant (PDA), and a tablet personal computer (PC).

The ultrasound data acquisition unit 110 may obtain ultrasound volume data with respect to the object 10 including the blood vessel 200 of FIG. 1. In an embodiment, the ultrasound data acquisition unit 110 may obtain 3D ultrasound volume data with respect to a carotid artery.

The term "ultrasound volume data" may refer to data consisting of a plurality of voxels. The ultrasound volume data with respect to the object 10 may include spatial information about the object 10 or clinical information, e.g., information about anatomical configurations of tissues or organs included in the object 10.

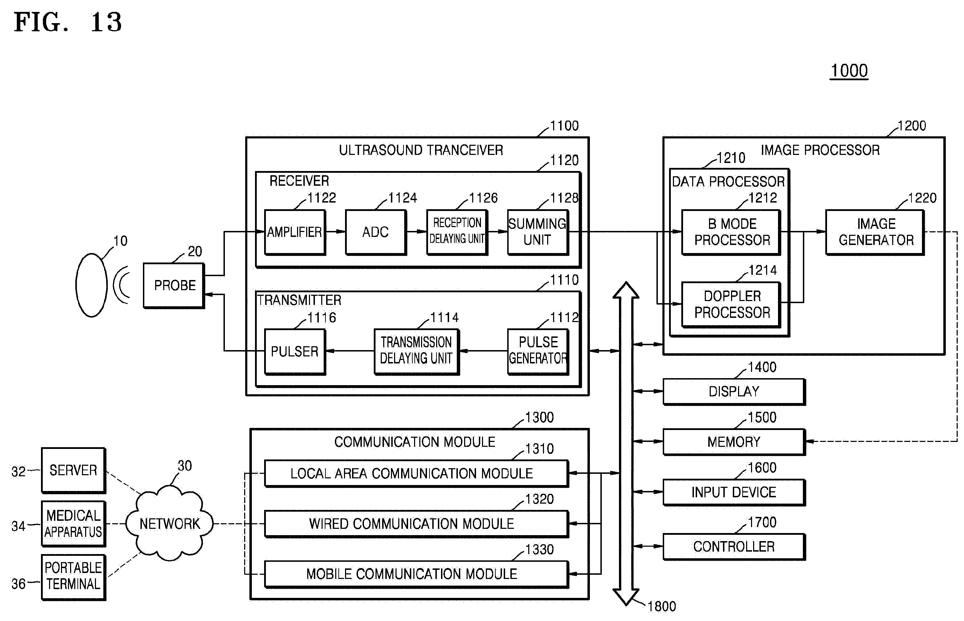

In an embodiment, the ultrasound data acquisition unit 110 may generate 3D ultrasound volume data with respect to the object 10 using the ultrasound data obtained from the object 10. In some embodiments, the ultrasound data acquisition unit 110 may obtain 3D ultrasound volume data from an external device via a communication module (see FIG. 13).

In an embodiment, the ultrasound data acquisition unit 110 may include the ultrasound probe 112 of FIG. 1 that directly transmits or receives ultrasound to or from the object 10. The ultrasound probe 112 may transmit ultrasound signals from the object 10 and receive echo signals from the object 10. The ultrasound probe 112 may transmit ultrasound signals to the object 10 in response to a driving signal applied to the ultrasound probe 112 and may receive echo signals reflected by the object 10.

The ultrasound probe 112 includes a plurality of transducers, and the plurality of transducers oscillate in response to electric signals and generate acoustic energy, that is, ultrasound waves. Furthermore, the ultrasound probe 112 may be connected to the main body of the ultrasound imaging apparatus 100 by wire or wirelessly, and according to embodiments, the ultrasound imaging apparatus 100 may include a plurality of ultrasound probes 112. In an embodiment, the ultrasound data acquisition unit 110 may include a 3D probe.

In an embodiment, the ultrasound probe 112 may transmit ultrasound plane waves to the object 10 and may receive ultrasound echo signals reflected by the object 10, based on the transmitted ultrasound signals.

The ultrasound data acquisition unit 110 may further include a data generator 114 that obtains ultrasound data from ultrasound echo signals received from the ultrasound probe 112.

In some embodiments, the ultrasound data acquisition unit 110 may receive ultrasound data with respect to the object 10 from an external source. The ultrasound data acquisition unit 110 may generate ultrasound data from an external apparatus connected to the ultrasound imaging apparatus 100 by wire or wirelessly.

The processor 120 may be at least one of a central processing unit (CPU), a graphic processing unit (GPU), and a micro processor. In an embodiment, the processor 120 may consist of hardware, such as FPGA or ASIC.

In an embodiment, the processor 120 may generate a 3D ultrasound image with respect to the object 10 by performing rendering with respect to 3D ultrasound volume data provided from the ultrasound data acquisition unit 110. The term 'rendering' as used herein denotes generating and displaying 2D projection image of a 3D discretely sampled data set, such as 3D ultrasound volume data. In an embodiment, the processor 120 may perform rendering using ray casting projection.

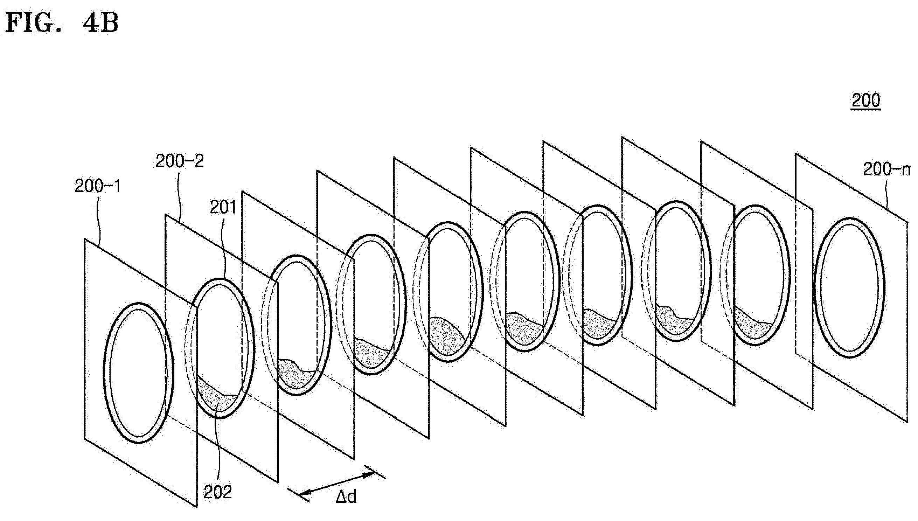

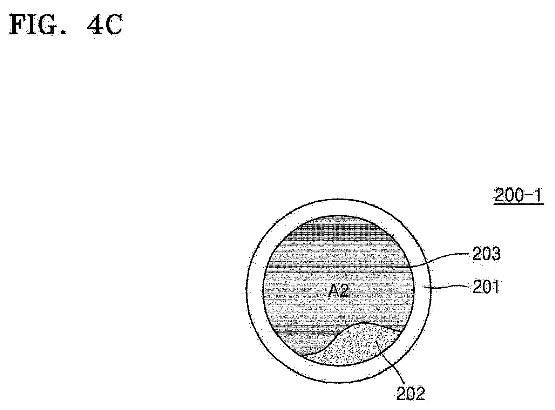

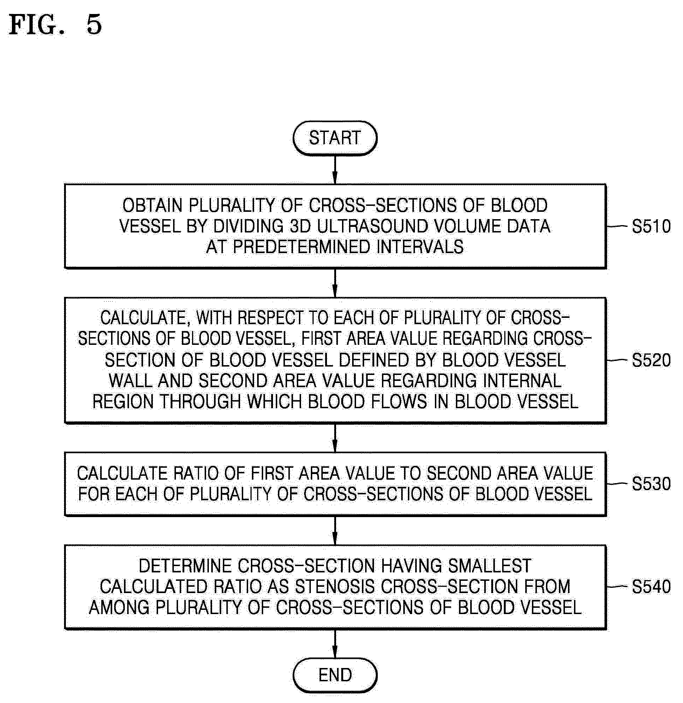

The processor 120 may analyze a degree of stenosis of a plurality of cross-sections of the blood vessel 200 included in the object 10 from the ultrasound data acquisition unit 110 and may determine a stenosis cross-section with the a highest degree of stenosis from among the plurality of cross-sections. In an embodiment, the processor 120 may obtain a plurality of blood vessel cross-sections by segmenting 3D ultrasound volume data with respect to the blood vessel 200 at constant intervals. In an embodiment, the processor 120 may calculate, with respect to each of the plurality of cross-sections, a first area value regarding a cross-section defined by a blood vessel wall of the blood vessel 200 and a second area value regarding a cross-section through which blood flows in the blood vessel 200; and may calculate, with respect to each of the plurality of cross-sections of the blood vessel 200, a ratio of the first area value to the second area value. The processor 120 may determine a cross-section having the smallest calculated ratio as a stenosis cross-section from among a plurality of blood vessel cross-sections. Methods of determining a stenosis cross-section will be described in detail in relation to FIGS. 4B, 4C, and 5.

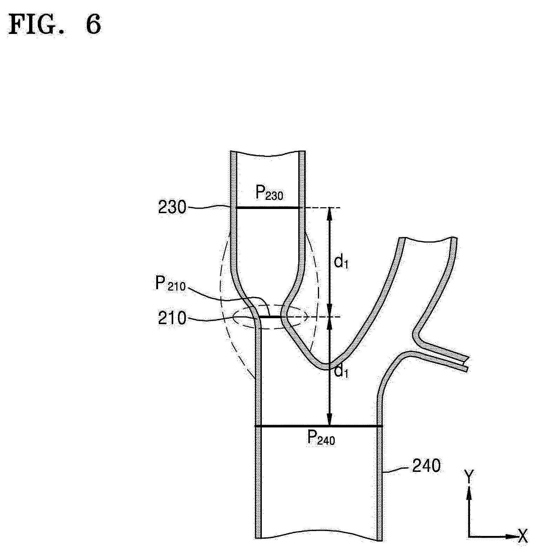

The processor 120 may determine a first cross-section and a second cross-section that are spaced apart from each other in different directions from the stenosis cross-section from among a plurality of cross-sections of the blood vessel 200. In an embodiment, the processor 120 may determine a first cross-section and a second cross-section that are spaced apart by the same interval in opposite directions from the stenosis cross-section. In an embodiment, the processor 120 may detect a branch point where the blood vessel 200 diverges into two from the 3D ultrasound volume data with respect to the blood vessel obtained from the ultrasound data acquisition unit 110 and may determine a first cross-section and a second cross-section spaced apart by the same interval on different directions from the detected branch point. The method of determining a first cross-section and a second cross-section by the processor 120 will be described in detail in relation to FIGS. 6 to 10.

The processor 120 may calculate at least two stenosis index values, which numerically represent a degree of stenosis of the blood vessel 200, from a diameter or a cross-sectional area of each of the determined stenosis cross-section, first cross-section, and second cross-section. In an embodiment, the processor 120 may calculate at least two index values, which may be selected from NASCET, ECST, and CC. The stenosis index values will be described in detail in relation to FIG. 11.

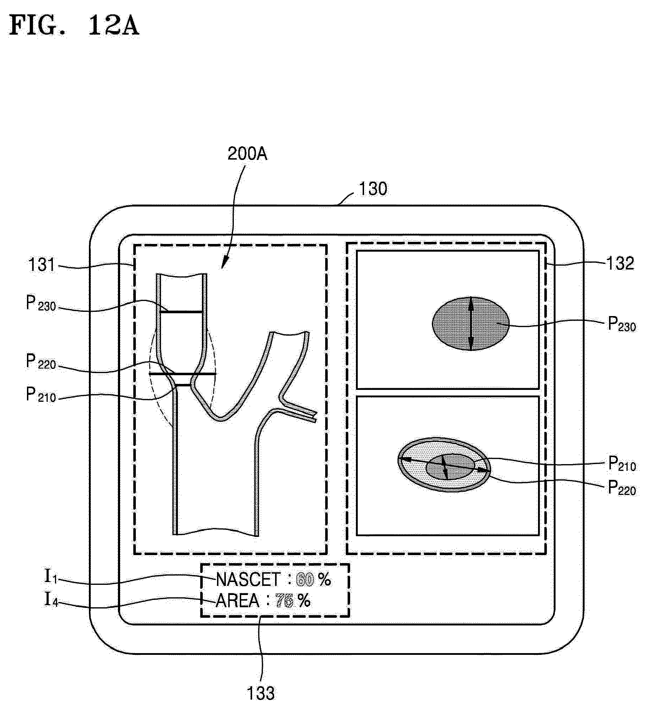

The display unit 130 may display at least two cross-sections of the stenosis cross-section, the first cross-section, and the second cross-section and at least two stenosis index values. In an embodiment, the display unit 130 may display a 3D ultrasound image with respect to the object 10 alongside the stenosis cross-section, the first cross-section, the second cross-section, and a plurality of stenosis index values. In an embodiment, the display unit 130 may display a plurality of stenosis index values along with a user interface that displays at least one of different colors, graphs, and figures, based on sizes of at least two stenosis index values. Displaying at least two stenosis index values on the display unit 130 will be described in detail in relation to FIGS. 12A to 12D.

The ultrasound imaging apparatus 100 according to an embodiment may determine a stenosis cross-section of the blood vessel 200 with a highest degree of stenosis from 3D ultrasound volume data of the blood vessel 200 of FIG. 1 included in the object 10, determine a first cross-section and a second cross-section that are spaced apart in different directions from the stenosis cross-section of the blood vessel 200, and may calculate at least two stenosis index values representing a degree of stenosis of the blood vessel 200, based on a diameter or a cross-section area size of the stenosis cross-section, the first cross-section, and the second cross-section. In addition, the ultrasound imaging apparatus 100 may display the stenosis cross-section, the first cross-section, and the second cross-section and the at least two stenosis index values on the display unit 130, improving diagnostic usability for the user. Furthermore, the ultrasound imaging apparatus 100 may display at least two stenosis index values by using at least one of different colors, graphs, and figures, enabling a user to quickly and accurately recognize the degree of stenosis of a blood vessel.

The method of determining a stenosis cross-section, a first cross-section, and a second cross-section and the method of displaying at least two stenosis index values in the ultrasound imaging apparatus 100, according to an embodiment, will be described in detail hereinafter.

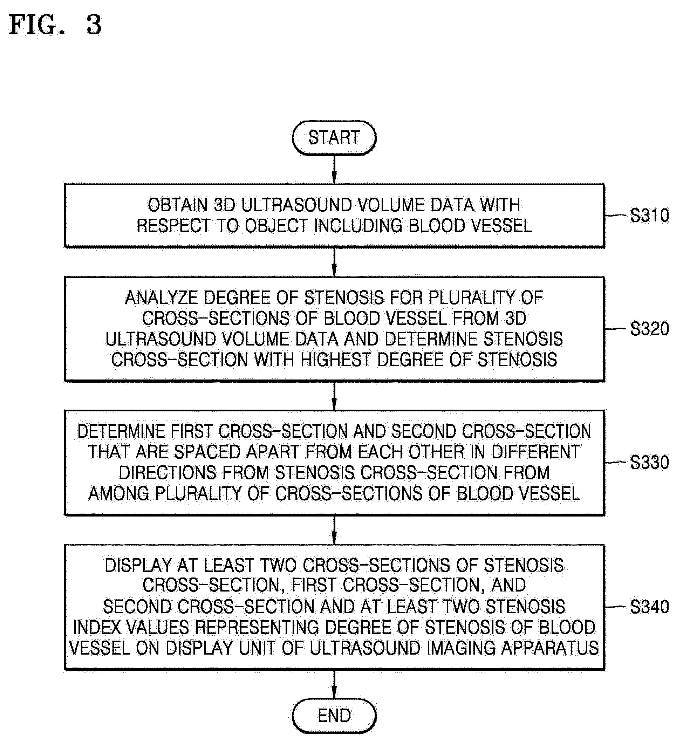

FIG. 3 is a flowchart of a method, performed by the ultrasound imaging apparatus 100, of displaying a plurality of blood vessel cross-sections related to measurement of a degree of stenosis of a blood vessel and stenosis index values, according to an embodiment.

In operation S310, the ultrasound imaging apparatus 100 may obtain 3D ultrasound volume data with respect to an object including a blood vessel. In an embodiment, the ultrasound imaging apparatus 100 may include an ultrasound probe, and the ultrasound probe may transmit ultrasound signals to an object and may receive ultrasound echo signals reflected by the object. The ultrasound imaging apparatus 100 may obtain 3D ultrasound volume data from the received ultrasound echo signals.

In operation S320, the ultrasound imaging apparatus 100 may analyze a degree of stenosis for a plurality of blood vessel cross-sections from 3D ultrasound volume data and determine a stenosis cross-section with a highest degree of stenosis. In an embodiment, the ultrasound imaging apparatus 100 may obtain a plurality of blood vessel cross-sections by segmenting 3D ultrasound volume data with respect to the blood vessel at constant intervals. The ultrasound imaging apparatus 100 may calculate, with respect to each of the plurality of blood vessel cross-sections, a ratio of an area value of a cross-section of the entire blood vessel defined by the blood vessel wall to an area value of a cross-section through which blood flows in the blood vessel and determine a cross-section having the smallest calculated ratio as a stenosis cross-section.

In operation S330, the ultrasound imaging apparatus 100 may determine a first cross-section and a second cross-section that are spaced apart from each other in different directions from the stenosis cross-section from among a plurality of blood vessel cross-sections. In an embodiment, the ultrasound imaging apparatus 100 may determine a first cross-section and a second cross-section that are spaced apart by the same interval in opposite directions from the stenosis cross-section determined in operation S320.

In operation S340, the ultrasound imaging apparatus 100 may display at least two cross-sections of the stenosis cross-section, the first cross-section, and the second cross-section and at least two stenosis index values representing the degree of stenosis of the blood vessel. In an embodiment, the ultrasound imaging apparatus 100 may calculate the at least two stenosis index values representing a degree of stenosis of the blood vessel, based on a diameter or a cross-section area of the stenosis cross-section, the first cross-section, and the second cross-section. In an embodiment, the ultrasound imaging apparatus 100 may render 3D ultrasound volume data with respect to the object obtained in operation S310 to generate a 3D ultrasound image with respect to the blood vessel and display the generated 3D ultrasound image alongside the stenosis cross-section, the first cross-section, the second cross-section, and the at least two stenosis index values on the display unit 130 in FIGS. 1 and 2.

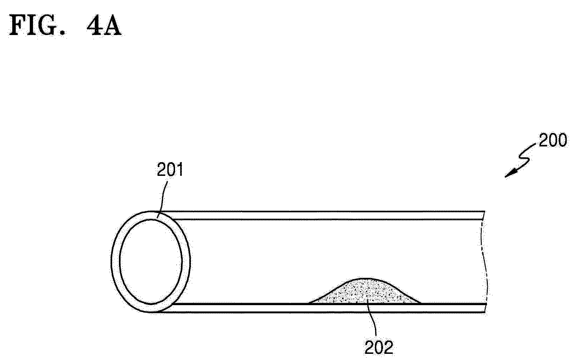

FIG. 4A is a diagram for explaining a structure of a blood vessel that is stenosed.

Referring to FIG. 4A, the ultrasound imaging apparatus 100 may generate and display an ultrasound image of the blood vessel 200, e.g., a carotid artery. The image generated by the ultrasound imaging apparatus 100 may include plaque 202 deposited on and accumulated in the blood vessel wall 201. The image displayed on a display of the ultrasound imaging apparatus 100, which shows the plaque 202, may have a variation in at least one of a size, a shape, and a position of the plaque 202, depending on a degree of stenosis of the blood vessel. Depending on the size, the shape, and the position of the plaque 202 deposited in the blood vessel 200, the size of region through which the blood can actually flow in the entire area of the blood vessel defined by the blood vessel wall 201 may vary.

FIG. 4B is a diagram for explaining a method, performed by the ultrasound imaging apparatus 100, of obtaining a plurality of blood vessel cross-sections from 3D ultrasound volume data, according to an embodiment.

Referring to FIG.4B, the ultrasound imaging apparatus 100 may obtain a plurality of blood vessel cross-sections 200-1 to 200-n from 3D ultrasound volume data with respect to the blood vessel 200. In an embodiment, the ultrasound imaging apparatus 100 may obtain the plurality of blood vessel cross-sections 200-1 to 200-n by segmenting 3D ultrasound volume data with respect to the blood vessel 200 at predetermined intervals $\Delta d$. The plurality of blood vessel cross-sections 200-1 to 200-n may each include the blood vessel wall 201 and the plaque 202. The plaque 202 included in the plurality of blood vessel cross-sections 200-1 to 200-n may vary in size and shape.

The ultrasound imaging apparatus 100 may determine a cross-section, which may have the largest area ratio of the entire area of the blood vessel defined by the blood vessel wall 201 to plaque 202, as a stenosis cross-section from among the plurality of blood vessel cross-sections 200-1 to 200-n. The degree of stenosis of a cross-section of the blood vessel 200 may mean the degree of constriction of the blood vessel 200 due to accumulation of deposited plaque on the blood vessel wall 201. The degree of stenosis of a cross-section of the blood vessel 200 may be measured by comparing an area of a reference region defined by the blood vessel wall 201 with an area of an internal region through which the blood flows. The internal region may mean an area of a blood vessel through which the blood actually flows with the exception of the plaque 202 accumulated on the blood vessel wall 201. The ultrasound imaging apparatus 100 may deduct the cross-section area, through which the blood flows, from the reference region to thereby measure a ratio of the plaque 202 accumulated on the blood vessel wall.

The method, performed by the ultrasound imaging apparatus 100, of determining a stenosis cross-section from among the plurality of blood vessel cross-sections 200-1 to 200-n will be described with reference to FIG. 4C.

FIG. 4C is a diagram for explaining a method, performed by the ultrasound imaging apparatus 100, of calculating a degree of stenosis for a plurality of blood vessel cross-sections, according to an embodiment.

FIG. 4C is a cross-sectional view of a cross-section 200-1 from among the plurality of blood vessel cross-sections 200-1 to 200-n. The blood vessel wall 201, the plaque 202, and an internal region 203 through which the blood flows in the blood vessel 200 are depicted in FIG. 4C. The ultrasound imaging apparatus 100 may obtain a brightness value from ultrasound data for the plurality of blood vessel cross-sections 200-1 to 200-n in FIG. 4B. In an embodiment, the ultrasound imaging apparatus 100 may determine an area of a region where the obtained brightness value is less than a predetermined brightness value as the internal region 203.

The ultrasound imaging apparatus 100 may calculate a first area value regarding the entire region defined by the blood vessel wall 201, and calculate a second area value regarding an area A2 of the internal region 203 through which the blood flows excluding the plaque 202. The ultrasound imaging apparatus 100 may calculate a first area value and a second area value with respect to each of the plurality of blood vessel cross-sections 200-1 to 200-n and a ratio of the first area value to the second area value with respect to each of the plurality of blood vessel cross-sections 200-1 to 200-n.

The ultrasound imaging apparatus 100 may determine a cross-section having the smallest calculated ratio with respect to the plurality of blood vessel cross-sections 200-1 to 200-n as a stenosis cross-section. In other words, a blood vessel cross-section having the smallest ratio of the plaque 202 in the cross-sectional area of the entire blood vessel defined by the blood vessel wall 201 is determined as a stenosis cross-section.

FIG. 5 is a flowchart of a method, performed by the ultrasound imaging apparatus 100, of determining a stenosis cross-section from among a plurality of cross-sections of a blood vessel, according to an embodiment.

In operation S510, the ultrasound imaging apparatus 100 may obtain a plurality of blood vessel cross-sections by segmenting 3D ultrasound volume data with respect to an object including a blood vessel at constant intervals.

In operation S520, the ultrasound imaging apparatus 100 may calculate, with respect to each of the plurality of cross-sections of the blood vessel, a first area value regarding a cross-section of the blood vessel defined by a blood vessel wall and a second area value regarding an internal region through which the blood flows in the blood vessel. The blood vessel may include plaque deposited on the blood vessel wall, and the second area value for the plurality of cross-sections of the blood vessel may vary due to the difference in size and shape of the plaque.

The ultrasound imaging apparatus 100 may obtain a brightness value from ultrasound volume data with respect to each of the plurality of cross-sections of the blood vessel and determine an area of a region where the obtained brightness value is less than a predetermined brightness value as the internal region. The ultrasound imaging apparatus 100 may calculate the second area value regarding the determined internal region.

In operation S530, the ultrasound imaging apparatus 100 may calculate a ratio of the first area value to the second area value for each of the plurality of blood vessel cross-sections.

In operation S540, the ultrasound imaging apparatus 100 may determine a cross-section having the smallest calculated ratio as a stenosis cross-section from among the plurality of blood vessel cross-sections.

FIG. 6 is a diagram for explaining a method, performed by the ultrasound imaging apparatus 100, of determining the plurality of blood vessel cross-sections $P_{230}$ and $P_{240}$ for measurement of a degree of stenosis of the blood vessel 200, according to an embodiment. In an embodiment, the blood vessel 200 may be a carotid artery, and a stenosis section 210 may be formed between an internal carotid artery 230 and a common carotid artery 240.

Referring to FIG. 6, the ultrasound imaging apparatus 100 may determine the stenosis cross-section $P_{210}$ from 3D ultrasound volume data with respect to the blood vessel 200 and determine the first cross-section $P_{230}$ and the second cross-section $P_{240}$ spaced apart from each other in different directions from the stenosis cross-section $P_{210}$. The ultrasound imaging apparatus 100 may determine the stenosis cross-section $P_{210}$ from among the plurality of blood vessel cross-sections 200-1 to 200-n in FIG. 4B by using the method described with reference to FIGS. 4B, 4C, and 5, but embodiments are not limited thereto. In an embodiment, the ultrasound imaging apparatus 100 may receive a user input for setting the stenosis cross-sections $P_{210}$ from among the plurality of blood vessel cross-sections 200-1 to 200-n and determine the stenosis cross-sections $P_{210}$ based on the user input.

In an embodiment, the ultrasound imaging apparatus 100 may determine the first cross-section $P_{230}$ spaced apart by a predetermined first interval d1 in the first direction Y from the determined stenosis cross-section $P_{210}$ and the second cross-section $P_{240}$ spaced apart by the first interval d1 in a second direction opposite to the first direction Y from the stenosis cross-section $P_{210}$. In an embodiment, the first interval d1 may be 1 cm or less, but embodiments are not limited thereto. The ultrasound imaging apparatus 100 may automatically determine the first cross-section $P_{230}$ and the second cross-section $P_{240}$, once the determination of the stenosis cross-section $P_{210}$ is complete. However, embodiments are not limited thereto; and the ultrasound imaging apparatus 100 may receive a user input for setting the first cross-section $P_{230}$ and the second cross-section $P_{240}$ and determine the first cross-section $P_{230}$ and the second cross-section $P_{240}$ based on the received user input.

The first interval d1 may be predetermined, embodiments are not limited thereto. In an embodiment, the ultrasound imaging apparatus 100 may receive a user input for setting the first interval d1 and determine the first interval d1 based on the received user input.

In an embodiment, the blood vessel 200 may be a carotid artery, the first cross-section $P_{230}$ determined by the ultrasound imaging apparatus 100 may be a cross-section of an internal carotid artery (ICA), and the second cross-section $P_{24}$ may be a cross-section of a common carotid artery (CCA). However, these are for illustrative purposes only; and the blood vessel 200 is not limited thereto.

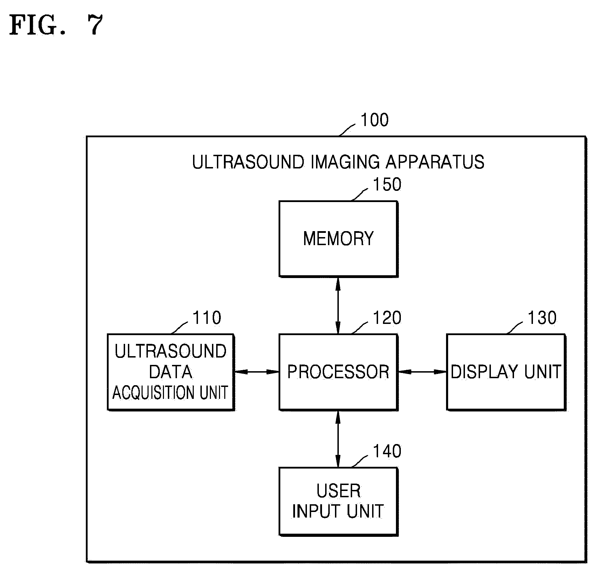

FIG. 7 is a block diagram showing a configuration of an ultrasound imaging apparatus 100 according to another embodiment.

Referring to FIG. 7, the ultrasound imaging apparatus 100 may include the ultrasound data acquisition unit 110, the processor 120, the display unit 130, a user input unit 140, and a memory 150. The ultrasound data acquisition unit 110, the processor 120, and the display unit 130 may be the same as those described above in relation to FIG. 2; and thus descriptions thereof will be omitted here.

The user input unit 140 may receive a user input for controlling the ultrasound imaging apparatus 100. For example, the user input unit 140 may include hardware components, such as a keypad, a mouse, a touch pad, a touch screen, a track ball, and a jog switch. However, embodiments are not limited thereto. In an embodiment, the user input unit 140 may receive a user input for setting the stenosis cross-section $P_{210}$ in FIG. 6 from among a plurality of blood vessel cross-sections of 3D ultrasound volume data obtained from the ultrasound data acquisition unit 110. Furthermore, in an embodiment, the user input unit 140 may receive a user input for setting the first interval d1 in FIG. 6 spaced apart from the stenosis cross-section $P_{210}$ for determination of the first and second cross-sections $P_{230}$ and $P_{240}$ in FIG. 6. In addition, in an embodiment, the user input unit 140 may receive a user input for setting the first cross-section $P_{230}$ and the second cross-section $P_{240}$.

The memory 150 stores various data processed by the ultrasound imaging apparatus 100. The memory 1500 may be any of various storage media, e.g., a flash memory, a hard disk drive, EEPROM, etc, but embodiments are not limited thereto. In an embodiment, the memory 150 may store information about the stenosis cross-section $P_{210}$, the first cross-section $P_{230}$, and the second cross-section $P_{240}$ according to a user input received from the user input unit 140. Additionally, in an embodiment, the memory 150 may store a value of the first interval d1 received from the user input unit 140.

In an embodiment, the memory 150 may store a 3D ultrasound image generated by volume rendering in the processor 120.

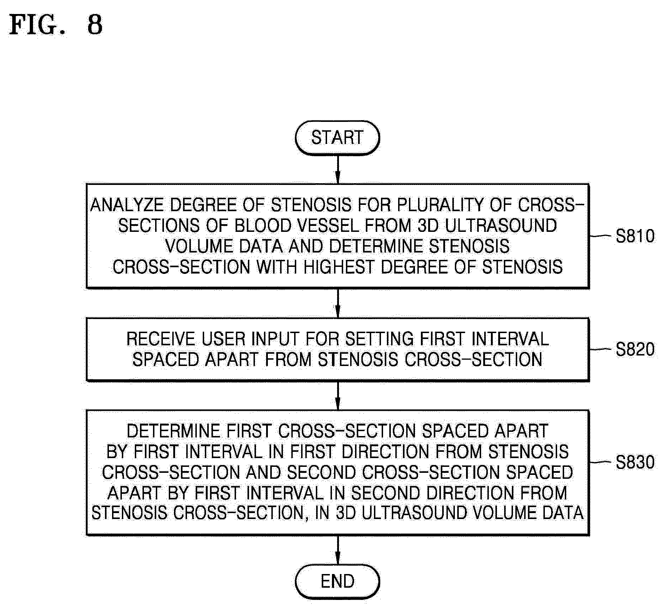

FIG. 8 is a flowchart of a method, performed by the ultrasound imaging apparatus 100, of determining a plurality of blood vessel cross-sections for measurement of a degree of stenosis of the blood vessel, according to an embodiment.

In operation S810, the ultrasound imaging apparatus 100 may analyze a degree of stenosis for a plurality of blood vessel cross-sections from 3D ultrasound volume data and determine a stenosis cross-section with a highest degree of stenosis. Operation S810 is the same as operation S320 described above in relation to FIG. 3, and thus descriptions thereof will be omitted here.

In operation S820, the ultrasound imaging apparatus 100 may receive a user input for setting a first interval spaced apart from the stenosis cross-section. The user input unit 140 in FIG. 7 of the ultrasound imaging apparatus 100 may receive a user input for setting a first interval and the received first interval value may be stored in the memory 150 in FIG. 7.

In operation S830, the ultrasound imaging apparatus 100 may determine a first cross-section spaced apart by the first interval in a first direction from the stenosis cross-section and a second cross-section spaced apart by the first interval in a second direction from the stenosis cross-section, in 3D ultrasound volume data. The first cross-section and the second cross-section may be spaced apart in opposite directions from each other. That is, the second direction may be opposite to the first direction. When a stenosis cross-section is determined in operation S810, the ultrasound imaging apparatus 100 may automatically determine a first cross-section and a second cross-section based on a user input for setting the first interval, but embodiments are not limited thereto. In an embodiment, the ultrasound imaging apparatus 100 may receive a user input for setting the first cross-section and the second cross-section and determine the first cross-section and the second cross-section based on the received user input.

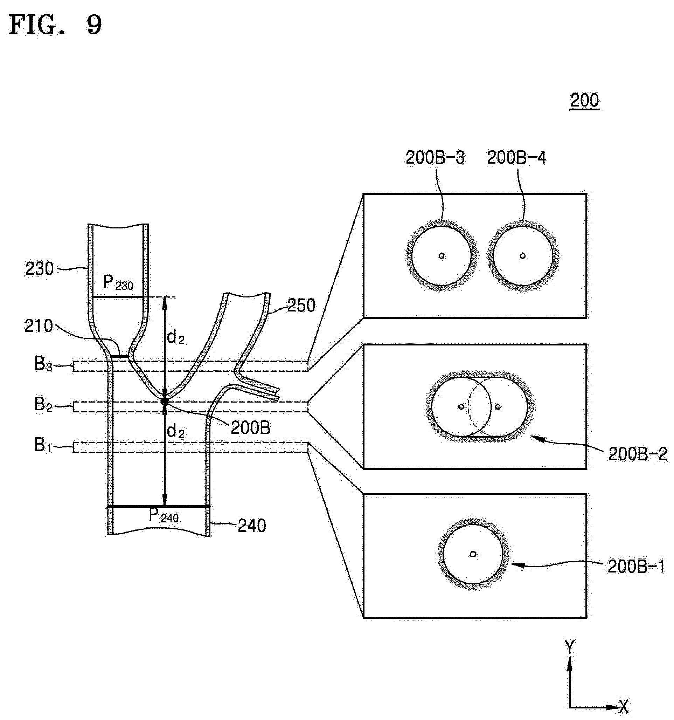

FIG. 9 is a diagram for explaining a method, performed by the ultrasound imaging apparatus 100, of determining a plurality of blood vessel cross-sections for measurement of a degree of stenosis of the blood vessel 200, according to an embodiment.

Referring to FIG. 9, the ultrasound imaging apparatus 100 may obtain the plurality of blood vessel cross-sections 200-1 to 200-4 from 3D ultrasound volume data with respect to the blood vessel 200 and determine the first cross-section $P_{230}$ and the second cross-section $P_{240}$ from among the plurality of blood vessel cross-sections 200-1 to 200-4.

The ultrasound imaging apparatus 100 may analyze the shape of the blood vessel 200 in the plurality of blood vessel cross-sections 200-1 to 200-4 to detect a branch point 200B where the blood vessel 200 diverges into two. In an embodiment, the ultrasound imaging apparatus 100 may track cross-sections of the blood vessel 200 from the lower side in a Y direction to analyze the shape of the blood vessel 200. For example, the ultrasound imaging apparatus 100 may detect a first point cross-section 200B-1 with respect to the blood vessel 200 at a first point $B_1$. The first point cross-section 200B-1 may mean a cross-section of the blood vessel 200 in an X direction at the first point $B_1$ from among a plurality of points segmented at constant intervals in the Y direction. Likewise, the ultrasound imaging apparatus 100 may detect a second point cross-section 200B-2 with respect to the blood vessel 200 at a second point $B_2$ and third point cross-sections 200B-3 and 200B-4 with respect to the blood vessel 200 at a third point $B_3$. The ultrasound imaging apparatus 100 may detect the third point cross-sections 200B-3 and 200B-4, which may be blood vessel cross-sections that are divided at the third point $B_3$. As shown in FIG. 9, the ultrasound imaging apparatus 100 may detect a branch point 200B where the blood vessel 200 diverges into two branches, in which the branch point 200B may be between the second point $B_2$ and the third point $B_3$.

The ultrasound imaging apparatus 100 may determine, about the branch point 200B, the first cross-section $P_{230}$ spaced apart by a second interval $d_2$ in the first direction Y and the second cross-section $P_{240}$ spaced apart by the second interval $d_2$ in a second direction opposite to the first direction Y. In an embodiment, the second interval $d_2$ may be predetermined. The second interval $d_2$ may be stored in the memory 150. In an embodiment, the ultrasound imaging apparatus 100 may receive a user input for setting the second interval $d_2$ and determine the second interval $d_2$ based on the received user input. In an embodiment, the second interval $d_2$ may have a value of about 1 cm or less, but embodiments are not limited thereto.

In an embodiment, the blood vessel 200 may be a carotid artery, the first cross-section $P_{230}$ determined by the ultrasound imaging apparatus 100 may be a cross-section of an ICA, and the second cross-section $P_{24}$ may be a cross-section of a CCA. However, these are for illustrative purposes only; and the blood vessel 200 is not limited thereto.

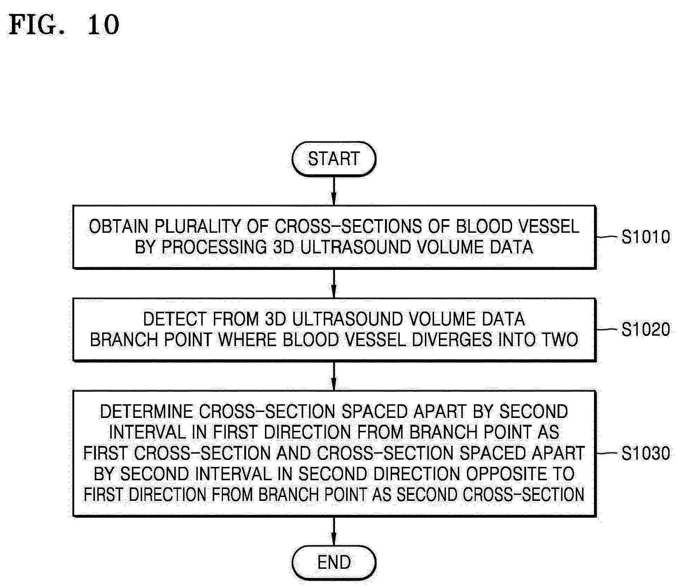

FIG. 10 is a flowchart of a method, performed by the ultrasound imaging apparatus 100, of determining a plurality of blood vessel cross-sections for measurement of a degree of stenosis of the blood vessel, according to an embodiment.

In operation S1010, the ultrasound imaging apparatus 100 may obtain a plurality of blood vessel cross-sections by processing 3D ultrasound volume data with respect to an object including a blood vessel. Operation S1010 is the same as operation S320 explained in relation to FIG. 3, and thus descriptions thereof will be omitted.

In operation S1020, the ultrasound imaging apparatus 100 may detect from the 3D ultrasound volume data a branch point where the blood vessel diverges into two. The ultrasound imaging apparatus 100 may obtain, from the 3D ultrasound volume data, a plurality of blood vessel cross-sections that are segmented at constant intervals in the first direction Y at a plurality of points. The ultrasound imaging apparatus 100 may analyze the plurality of blood vessel cross-sections to detect a branch point where the blood vessel diverges into two. In an embodiment, the ultrasound imaging apparatus 100 may detect the branch point by the change of the number of blood vessels included in the plurality of blood vessel cross-sections.

In operation S1030, the ultrasound imaging apparatus 100 may determine a cross-section spaced apart by a second interval in the first direction Y from the branch point detected in operation S1020 as a first cross-section and a cross-section spaced apart by the second interval in a second direction opposite to the first direction Y from the branch point as a second cross-section. The value of the second interval may be a predetermined value, but embodiments are not limited thereto. The ultrasound imaging apparatus 100 may receive a user input for setting a value of the second interval and determine the second interval based on the received user input.

FIG. 11A is a diagram for explaining a method, performed by the ultrasound imaging apparatus 100, of calculating a stenosis index value representing a degree of stenosis of the blood vessel 200, according to an embodiment. FIG. 11B is a cross-sectional view of the blood vessel 200 for explaining a method, performed by the ultrasound imaging apparatus 100, of calculating a degree of stenosis of the blood vessel 200, according to an embodiment. In FIGS. 11A and 11B, the blood vessel 200 may be a carotid artery, but embodiments are not limited thereto.

Referring to FIGS. 11A and 11B, the ultrasound imaging apparatus 100 may determine the stenosis cross-sections $P_{210}$ and $P_{220}$, the first cross-section $P_{230}$, and the second cross-section $P_{240}$ from 3D ultrasound volume data with respect to the blood vessel 200. The stenosis cross-sections $P_{210}$ and $P_{220}$ shown in FIG. 11B may include the first stenosis cross-section $P_{210}$ with respect to an internal region through which the blood flows in the blood vessel 200 and plaque, and the second stenosis cross-section $P_{220}$, which is a cross-section of the entire blood vessel defined by the blood vessel wall.

The ultrasound imaging apparatus 100 may calculate at least two of the plurality of stenosis index values $I_1$, $I_2$, $I_3$, and $I_4$ representing a degree of stenosis of the blood vessel 200 based on a diameter or a cross-section area value of each of the stenosis cross-sections $P_{210}$ and $P_{220}$, the first cross-section $P_{230}$, and the second cross-section $P_{240}$.

In particular, the ultrasound imaging apparatus 100 may calculate the first index value $I_1$ representing a ratio of a diameter $D_{230}$ of the first cross-section $P_{230}$ to a difference between the diameter $D_{230}$ of the first cross-section $P_{230}$ and a diameter $D_{210}$ of the first stenosis cross-section $P_{210}$, as in Equation 1.

Equation 1

$$I_1 = \frac{D_{230} - D_{210}}{D_{230}} \times 100$$

In an embodiment, the first index value $I_1$ may be a NASCET index value.

The ultrasound imaging apparatus 100 may calculate the second index value $I_2$ representing a ratio of a diameter $D_{220}$ of the second stenosis cross-section $P_{220}$ to a difference between the diameter $D_{220}$ of the second stenosis cross-section $P_{220}$ and the diameter $D_{210}$ of the first stenosis cross-section $P_{210}$, as in Equation 2.

Equation 2

$$I_2 = \frac{D_{220} - D_{210}}{D_{220}} \times 100$$

In an embodiment, the second index value $I_2$ may be an ECST index value.

The ultrasound imaging apparatus 100 may calculate the third index value $I_3$ representing a ratio of a diameter $D_{240}$ of the second cross-section $P_{240}$ to a difference between the diameter $D_{240}$ of the second cross-section $P_{240}$ and the diameter $D_{210}$ of the first stenosis cross-section $P_{210}$, as in Equation 3.

Equation 3

$$I_3 = \frac{D_{240} - D_{210}}{D_{240}} \times 100$$

In an embodiment, the third index value $I_3$ may be a CC index value.

The ultrasound imaging apparatus 100 may calculate the fourth index value $I_4$ representing a ratio of an area value $A_{220}$ of the second stenosis cross-section $P_{220}$ to an area value $A_{210}$ of the first stenosis cross-section $P_{210}$, as in Equation 4.

Equation 4

$$I_4 = \frac{A_{210}}{A_{220}} \times 100$$

The ultrasound imaging apparatus 100 may obtain brightness values of each of a plurality of blood vessel cross-sections obtained from 3D ultrasound volume data with respect to the blood vessel 200 and determine an area of a region where the obtained brightness value is less than a predetermined brightness value as the stenosis cross-section $P_{210}$ to calculate the fourth index value $I_4$. This has been described in detail in relation to FIGS. 4C and 5, and thus a repeated description thereof will be omitted here. In an embodiment, the fourth index value $I_4$ may be an area reduction index value.

FIGS. 12A to 12D are diagrams for explaining a method, performed by the ultrasound imaging apparatus 100, of displaying the plurality of blood vessel cross-sections $P_{210}$, $P_{220}$, $P_{230}$ and $P_{240}$ for measurement of a degree of stenosis of the blood vessel 200 and the plurality of stenosis index values $I_1$, $I_2$, $I_3$, and $I_4$ representing the degree of stenosis of the blood vessel 200, according to an embodiment.

Referring to FIG. 12A, the ultrasound imaging apparatus 100 may display the 3D ultrasound image 200A with respect to the blood vessel 200, the plurality of blood vessel cross-sections $P_{210}$, $P_{220}$, and $P_{230}$, and the plurality of stenosis index values $I_1$ and $I_4$ on the display unit 130. In an embodiment, the 3D ultrasound image 200A may be displayed on a first region 131 of the display unit 130, the stenosis cross-sections $P_{210}$ and $P_{220}$ and the first cross-section $P_{230}$ may be displayed on a second region 132 of the display unit 130, and the two stenosis index values $I_1$ and $I_4$ may be displayed on a third region 133 of the display unit 130.

In FIG. 12A, it is illustrated that the first stenosis cross-section $P_{210}$, the second stenosis cross-section $P_{220}$, and the first cross-section $P_{230}$ are all displayed on the second region 132, but embodiments are not limited thereto. At least two cross-sections of the first stenosis cross-section $P_{210}$, the second stenosis cross-section $P_{220}$, the first cross-section $P_{230}$, and the second cross-section $P_{240}$ may be displayed on the second region 132. In an embodiment, a user interface representing a diameter of each of the first stenosis cross-section $P_{210}$, the second stenosis cross-section $P_{220}$, and the first cross-section $P_{230}$ may be displayed on the second region 132.

In an embodiment, the 3D ultrasound image 200A may be an ultrasound image of a carotid artery and the first cross-section $P_{230}$ may be a cross-section of ICA.

In FIG. 12A, it is illustrated that the first index value $I_1$ and the fourth index value $I_4$ are displayed on the third region 133, but embodiments are not limited thereto. In an embodiment, at least two index values of the first index value $I_1$ as a NASCET value, the second index value $I_2$ as an ECST value, the third index value $I_3$ as a CC value, and the fourth index value $I_4$ as an area reduction value may be displayed on the third region 133. For example, three index values including the first index value $I_1$, the second index value $I_2$, and the third index value $I_3$ may be displayed on the third region 133.

In the embodiment illustrated in FIG. 12A, the ultrasound imaging apparatus 100 may display the stenosis cross-sections $P_{210}$ and $P_{220}$, the first cross-section $P_{230}$, and the two stenosis index values $I_1$ and $I_4$ representing a degree of stenosis of the blood vessel 200 on the display unit 130, enabling a user to easily recognize the degree of stenosis of the blood vessel 200 and improving diagnostic usability for the user. Furthermore, the ultrasound imaging apparatus 100 may display the 3D ultrasound image 200A with respect to the blood vessel 200 alongside the stenosis cross-sections $P_{210}$ and $P_{220}$, the first cross-section $P_{230}$, and the two stenosis index values $I_1$ and $I_4$ on the display unit 130, enabling a user to easily recognize a degree of stenosis in accordance with the structure of the blood vessel 200.

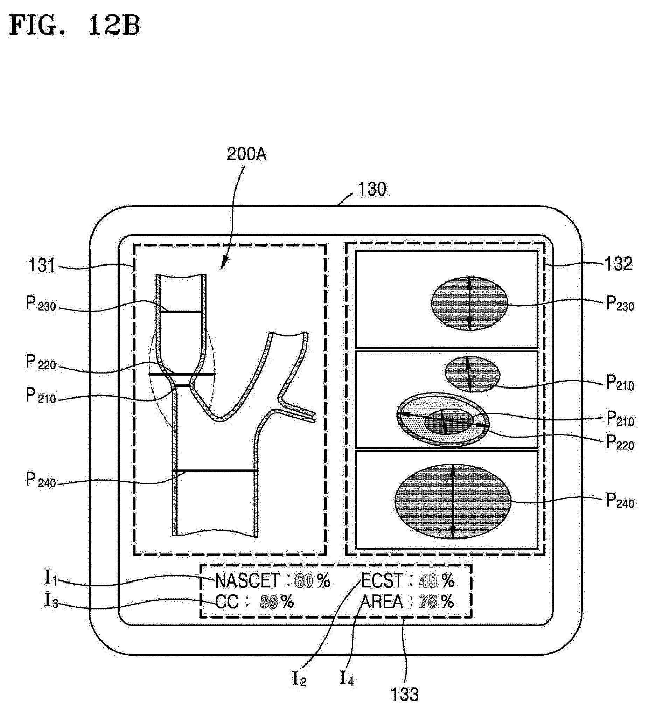

Referring to FIG. 12B, the ultrasound imaging apparatus 100 may display the 3D ultrasound image 200A with respect to the blood vessel 200, the plurality of blood vessel cross-sections $P_{210}$, $P_{220}$, $P_{230}$, and $P_{240}$ and the plurality of stenosis index values $I_1$, $I_2$, $I_3$, and $I_4$ on the display unit 130. In the embodiment illustrated in FIG. 12A, the four stenosis index values $I_1$, $I_2$, $I_3$, and $I_4$ are shown; however this is for illustrative purposes only and three stenosis index values of the four stenosis index values $I_1$, $I_2$, $I_3$, and $I_4$ may be displayed. In an embodiment, the ultrasound imaging apparatus 100 may select any three stenosis index values to be displayed on the display unit 130 from the four stenosis index values $I_1$, $I_2$, $I_3$, and $I_4$. The plurality of stenosis index values $I_1$, $I_2$, $I_3$, and $I_4$ to be displayed on the display unit 130 may be the same as those described in relation to FIG. 12A, and thus descriptions thereof will be omitted here.

In an embodiment, the plurality of stenosis index values $I_1$, $I_2$, $I_3$, and $I_4$ to be displayed on the third region 133 may be displayed in different colors depending on the size of each of the index values. For example, when the size of the first index value $I_1$ is in a range of 0% to 40%, the first index value $I_1$ may be displayed in blue; when the size thereof is in a range of 40% to 60%, the first index value $I_1$ may be displayed in yellow; and when the size thereof is in a range of 60% to 90%, the first

index value $I_1$ may be displayed in red. In such a manner, the first index value $I_1$ may be displayed in different colors depending on the size of the index value. However, the correspondences between the foregoing numerical values and the colors are merely examples for explaining color displaying according to a size of an index value, and thus, the scope of the present disclosure is not limited thereto.

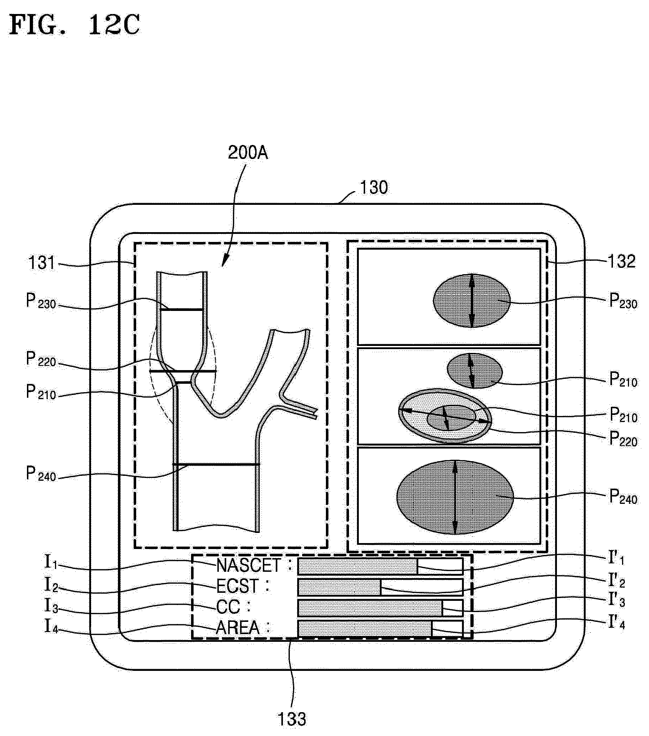

Referring to FIG. 12C, a user interface including a plurality of graphs $I_1'$, $I_2'$, $I_3'$, and $I_4'$ may be displayed on the third region 133 of the display unit 130, in which the plurality of graphs $I_1'$, $I_2'$, $I_3'$, and $I_4'$ each corresponds to the plurality of stenosis index values $I_1$, $I_2$, $I_3$, and $I_4$, respectively. The 3D ultrasound image 200A displayed on the first region 131 and the plurality of blood vessel cross-sections $P_{210}$, $P_{220}$, $P_{230}$, and $P_{240}$ displayed on the second region 132 may be the same as those described in connection with FIG. 12A, and thus, descriptions thereof will be omitted here.

The plurality of graphs $I_1'$, $I_2'$, $I_3'$, and $I_4'$ may each be distinctively displayed depending on a size of the plurality of stenosis index values $I_1$, $I_2$, $I_3$, and $I_4$, respectively. For example, when a size of the first index value $I_1$ is about 60%, the first graph $I_1'$ may be displayed at about 60% of the full size. Although bar graphs are shown in FIG. 12C, the type of the graphs are not limited thereto, and a pie chart, a graph of broken lines, or histogram. may also be used.

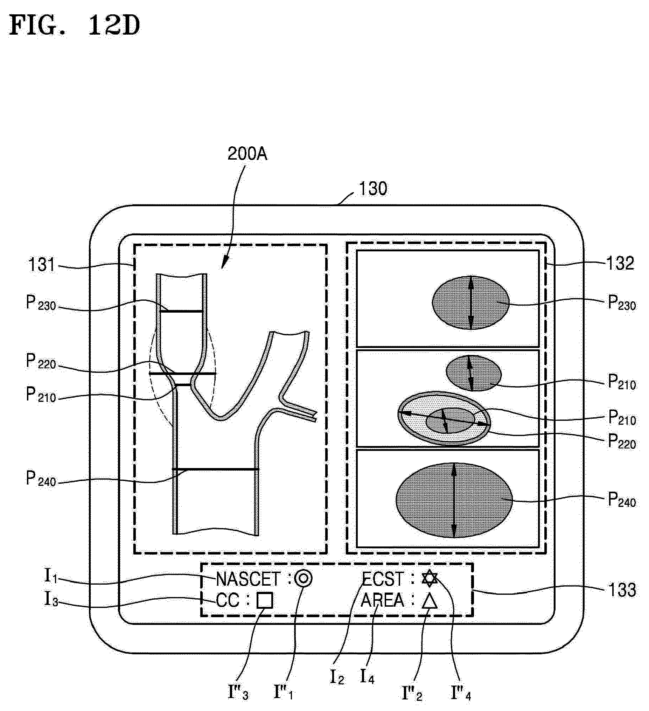

Referring to FIG. 12D, a user interface including a plurality of figures $I_1''$, $I_2''$, $I_3''$, and $I_4''$ each having distinct shapes may be displayed on the third region 133, in which the plurality of figures $I_1''$, $I_2''$, $I_3''$, and $I_4''$ respectively correspond to the plurality of stenosis index values $I_1$, $I_2$, $I_3$, and $I_4$. The 3D ultrasound image 200A displayed on the first region 131 and the plurality of blood vessel cross-sections $P_{210}$, $P_{220}$, $P_{230}$, and $P_{240}$ displayed on the second region 132 may be the same as those described in connection with FIG. 12A, and thus, descriptions thereof will be omitted here.

The plurality of figures $I_1''$, $I_1''$, $I_3''$, and $I_4''$ may each be distinctively displayed depending on a size of the plurality of stenosis index values $I_1$, $I_2$, $I_3$, and $I_4$, respectively. For example, when a size of the first index value $I_1$ is about 60%, and a size of the second index value $I_2$ is about 90%, the first figure $I_1'$ and the second figure I2' may have different shapes from each other. For example, the first figure $I_1''$ may be in a circular shape figure, and the second figure $I_2'$ may be in a star shape figure. However, figures shown in FIG. 12D are for illustrative purposes only; and the shapes of figures representing the plurality of stenosis index values $I_1$, $I_1$, $I_3$, and $I_4$ are not limited thereto.

In the embodiments of FIGS. 12C and 12D, the ultrasound imaging apparatus 100 may display graphs or figures for the plurality of stenosis index values $I_1$, $I_2$, $I_3$, and $I_4$, which represent a degree of stenosis of the blood vessel 200, enabling a user to intuitively recognize the degree of stenosis of the blood vessel 200.

FIG. 13 is a block diagram showing a configuration of an ultrasound diagnosis apparatus 1000 according to an embodiment. Referring to FIG. 13, the ultrasound diagnosis apparatus 1000 may include a probe 20, an ultrasound transceiver 1100, an image processor 1200, a communication module 1300, a display 1400, a memory 1500, an input device 1600, and a controller 1700, which may be connected to one another via buses 1800.

The ultrasound imaging apparatus 100 illustrated in FIG. 2 may include the same configuration as the ultrasound diagnosis apparatus 1000 illustrated in FIG. 13. In an embodiment, the ultrasound transceiver 1100 may be equivalent to the ultrasound data acquisition unit 110 shown in FIG. 2, the image processor 1200 may be equivalent to the processor 120 shown in FIG. 2, and the display 1400 may be equivalent to the display unit 130 shown in FIG. 2.

The ultrasound diagnosis apparatus 1000 may be a cart type apparatus or a portable type apparatus. Examples of portable ultrasound diagnosis apparatuses may include, but are not limited to, a PACS viewer, a smartphone, a laptop computer, a PDA, and a tablet PC.

The probe 20 transmits ultrasound waves to an object 10 in response to a driving signal applied by the ultrasound transceiver 1100 and receives echo signals reflected by the object 10. The probe 20 includes a plurality of transducers, and the plurality of transducers oscillate in response to electric signals and generate acoustic energy, that is, ultrasound waves. Furthermore, the probe 20 may be connected to the main body of the ultrasound diagnosis apparatus 1000 by wire or wirelessly, and according to embodiments, the ultrasound diagnosis apparatus 1000 may include a plurality of probes 20.

A transmitter 1110 supplies a driving signal to the probe 20. The transmitter 110 includes a pulse generator 1112, a transmission delaying unit 1114, and a pulser 1116. The pulse generator 1112 generates pulses for forming transmission ultrasound waves based on a pulse repetition frequency (PRF), and the transmission delaying unit 1114 delays the pulses by delay times necessary for determining transmission directionality. The pulses which have been delayed correspond to a plurality of piezoelectric vibrators included in the probe 20, respectively. The pulser 1116 applies a driving signal (or a driving pulse) to the probe 20 based on timing corresponding to each of the pulses which have been delayed.

A receiver 1120 generates ultrasound data by processing echo signals received from the probe 20. The receiver 120 may include an amplifier 1122, an analog-to-digital converter (ADC) 1124, a reception delaying unit 1126, and a summing unit 1128. The amplifier 1122 amplifies echo signals in each channel, and the ADC 1124 performs analog-to-digital conversion with respect to the amplified echo signals. The reception delaying unit 1126 delays digital echo signals output by the ADC 124 by delay times necessary for determining reception directionality, and the summing unit 1128 generates ultrasound data by summing the echo signals processed by the reception delaying unit 1166. In some embodiments, the receiver 1120 may not include the amplifier 1122. In other words, if the sensitivity of the probe 20 or the capability of the ADC 1124 to process bits is enhanced, the amplifier 1122 may be omitted.

The image processor 1200 generates an ultrasound image by scan-converting ultrasound data generated by the ultrasound transceiver 1100. The ultrasound image may be not only a grayscale ultrasound image obtained by scanning an object in an amplitude (A) mode, a brightness (B) mode, and a motion (M) mode, but also a Doppler image showing a movement of an object via a Doppler effect. The Doppler image may be a blood flow Doppler image showing flow of blood (also referred to as a color Doppler image), a tissue Doppler image showing a movement of tissue, or a spectral Doppler image showing a moving speed of an object as a waveform.

A B mode processor 1212 included in a data processor 1210 extracts B mode components from ultrasound data and processes the B mode components. An image generator 1220 may generate an ultrasound image indicating signal intensities as brightness based on the extracted B mode components 1212.

Similarly, a Doppler processor 1214 included in the data processor 1210 may extract Doppler components from ultrasound data, and the image generator 1220 may generate a Doppler image indicating a movement of an object as colors or waveforms based on the extracted Doppler components.

According to an embodiment, the image generator 1220 may generate a 3D ultrasound image via volume-rendering with respect to volume data and may also generate an elasticity image by imaging deformation of the object 10 due to pressure. Furthermore, the image generator 1220 may display various pieces of additional information in an ultrasound image by using text and graphics. In addition, the generated ultrasound image may be stored in the memory 1500.

A display 1400 displays the generated ultrasound image. The display 1400 may display not only an ultrasound image, but also various pieces of information processed by the ultrasound diagnosis apparatus 1000 on a screen image via a graphical user interface (GUI). In addition, the ultrasound diagnosis apparatus 1000 may include two or more displays 1400 according to embodiments.

The communication module 1300 is connected to a network 30 by wire or wirelessly to communicate with an external device or a server. The communication module 1300 may exchange data with a hospital server or another medical apparatus in a hospital, which is connected thereto via a PACS. Furthermore, the communication module 1300 may perform data communication according to the digital imaging and communications in medicine (DICOM) standard.

The communication module 1300 may transmit or receive data related to diagnosis of an object, e.g., an ultrasound image, ultrasound data, and Doppler data of the object, via the network 30 and may also transmit or receive medical images captured by another medical apparatus, e.g., a computed tomography (CT) apparatus, a magnetic resonance imaging (MRI) apparatus, or an X-ray apparatus. Furthermore, the communication module 1300 may receive information about a diagnosis history or medical treatment schedule of a patient from a server and utilizes the received information to diagnose the patient. Furthermore, the communication module 1300 may perform data communication not only with a server or a medical apparatus in a hospital, but also with a portable terminal of a medical doctor or patient.

The communication module 1300 is connected to the network 30 by wire or wirelessly to exchange data with a server 32, a medical apparatus 34, or a portable terminal 36. The communication module 1300 may include one or more components for communication with external devices. For example, the communication module 1300 may include a local area communication module 1310, a wired communication module 1320, and a mobile communication module 1330.

The local area communication module 1310 refers to a module for local area communication within a predetermined distance. Examples of local area communication techniques according to an embodiment may include, but are not limited to, wireless LAN, Wi-Fi, Bluetooth, ZigBee, Wi-Fi Direct (WFD), ultra wideband (UWB), infrared data association (IrDA), Bluetooth low energy (BLE), and near field communication (NFC).

The wired communication module 1320 refers to a module for communication using electric signals or optical signals. Examples of wired communication techniques according to an embodiment may include communication via a twisted pair cable, a coaxial cable, an optical fiber cable, and an Ethernet cable.

The mobile communication module 1330 transmits or receives wireless signals to or from at least one selected from a base station, an external terminal, and a server on a mobile communication network. The wireless signals may be voice call signals, video call signals, or various types of data for transmission and reception of text/multimedia messages.

The memory 1500 stores various data processed by the ultrasound diagnosis apparatus 1000. For example, the memory 1500 may store medical data related to diagnosis of an object, such as ultrasound data and an ultrasound image that are input or output, and may also store algorithms or programs which are to be executed in the ultrasound diagnosis apparatus 1000.

The memory 1500 may be any of various storage media, e.g., a flash memory, a hard disk drive, EEPROM, etc. Furthermore, the ultrasound diagnosis apparatus 1000 may utilize web storage or a cloud server that performs the storage function of the memory 1500 online.

The input device 1600 refers to a means via which a user inputs data for controlling the ultrasound diagnosis apparatus 1000. The input device 1600 may include hardware components, such as a keypad, a mouse, a touch pad, a touch screen, and a jog switch. However, embodiments are not limited thereto, and the input device 1600 may further include any of various other input units including an electrocardiogram (ECG) measuring module, a respiration measuring module, a voice recognition sensor, a gesture recognition sensor, a fingerprint recognition sensor, an iris recognition sensor, a depth sensor, a distance sensor, etc.

The controller 1700 may control all operations of the ultrasound diagnosis apparatus 1000. In other words, the controller 1700 may control operations among the probe 20, the ultrasound transceiver 1100, the image processor 1200, the communication module 1300, the display 1400, the memory 1500, and the input device 1600 shown in FIG. 13.

All or some of the probe 20, the ultrasound transceiver 1100, the image processor 1200, the communication module 1300, the display 1400, the memory 1500, the input device 1600, and the controller 1700 may be implemented as software modules. Furthermore, at least one selected from the ultrasound transceiver 1100, the image processor 1200, and the communication module 1300 may be included in the controller 1700. However, embodiments of the present invention are not limited thereto.

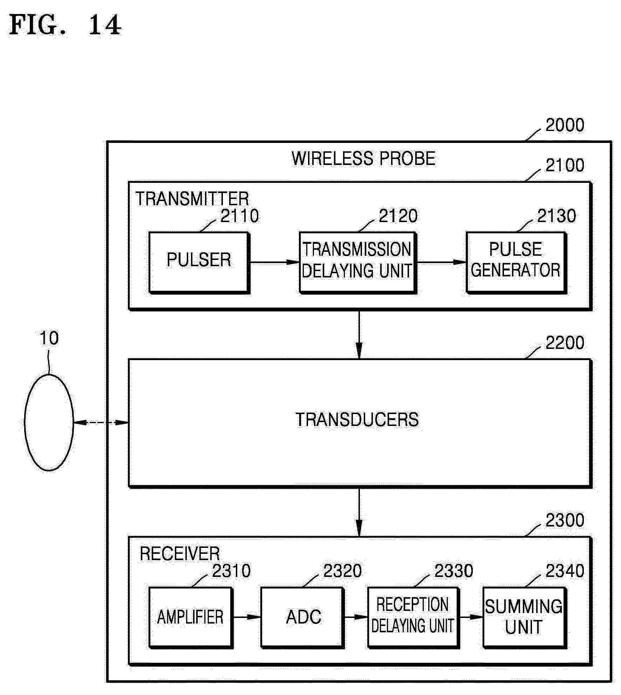

FIG. 14 is a block diagram showing a configuration of a wireless probe 2000 according to an embodiment. As described above with reference to FIG. 13, the wireless probe 2000 may include a plurality of transducers, and, according to embodiments, may include some or all of the components of the ultrasound transceiver 2100 shown in FIG. 13.

The wireless probe 2000 according to the embodiment shown in FIG. 2 includes a transmitter 2100, a transducer 2200, and a receiver 2300. Since descriptions thereof are given above with reference to FIG. 13, detailed descriptions thereof will be omitted here. In addition, according to embodiments, the wireless probe 2000 may selectively include a reception delaying unit 2330 and a summing unit 2340.

The wireless probe 2000 may transmit ultrasound signals to the object 10, receive echo signals from the object 10, generate ultrasound data, and wirelessly transmit the ultrasound data to the ultrasound diagnosis apparatus 1000 shown in FIG. 13.

The above-described embodiments of the present disclosure may be written as computer programs and may be implemented in general-use digital computers that execute the programs using a non-transitory computer-readable recording medium.

Examples of the non-transitory computer-readable recording medium include magnetic storage media (e.g., ROM, floppy disks, hard disks, etc.), optical recording media (e.g., CD-ROMs, or DVDs), etc), and transmission media such as Internet transmission media.

It should be understood that embodiments described herein should be considered in a descriptive sense only and not for purposes of limitation. Descriptions of features or aspects within each embodiment should typically be considered as available for other similar features or aspects in other embodiments.

While the present disclosure has been particularly shown and described with reference to exemplary embodiments thereof, it will be understood by those of ordinary skill in the art that various changes in form and details may be made therein without departing from the spirit and scope as defined by the following claims. Accordingly, the above embodiments and all aspects thereof are examples only and are not limiting.

**Claims**

1. A method, performed by an ultrasound imaging apparatus, of displaying an ultrasound image with respect to an object comprising a blood vessel, the method comprising:

   obtaining three-dimensional (3D) ultrasound volume data with respect to the object;
   analyzing a degree of stenosis for a plurality of cross-sections of the blood vessel from the 3D ultrasound volume data and determining a stenosis cross-section with a highest degree of stenosis;
   determining a first cross-section and a second cross-section that are spaced apart from each other in different directions from the stenosis cross-section from among the plurality of cross-sections of the blood vessel; and
   displaying at least two cross-sections of the stenosis cross-section, the first cross-section, the second cross-section, and at least two stenosis index values showing a degree of stenosis of the blood vessel on a display unit of the ultrasound imaging apparatus.

2. The ultrasound imaging method of claim 1, wherein the displaying of at least two stenosis index values comprises displaying at least two index values from among a first index value representing a ratio of a diameter of the first cross-section to a difference between the diameter of the first cross-section and a diameter of the stenosis cross-section, a second index value representing a ratio of an entire blood vessel diameter defined by a blood vessel wall of the stenosis cross-section to a difference between the entire blood vessel diameter and the diameter of the stenosis cross-section, a third index value representing a ratio of a diameter of the second cross-section to a difference between the diameter of the second cross-section and the diameter of the stenosis cross-section, and a fourth index value representing a ratio of a cross-sectional area of the entire blood vessel of the stenosis cross-section to an area of the stenosis cross-section.

3. The ultrasound imaging method of claim 1, wherein the displaying of the at least two cross-sections and the at least two stenosis index values comprises displaying a user interface that displays at least one of different colors, graphs, and figures based on sizes of the at least two stenosis index values, along with the at least two stenosis index values.

4. The ultrasound imaging method of claim 1, wherein the obtaining of the 3D ultrasound volume data with respect to the object comprises:

   transmitting ultrasound signals to the object;
   receiving ultrasound echo signals reflected by the object; and
   obtaining the 3D ultrasound volume data from the received ultrasound echo signals.

5. The ultrasound imaging method of claim 1, wherein the determining of the stenosis cross-section comprises:

   obtaining the plurality of cross-sections of the blood vessel by segmenting the 3D ultrasound volume data at predetermined intervals;
   calculating, with respect to each of the plurality of cross-sections of the blood vessel, a first area value regarding a cross-section defined by a blood vessel wall of the blood vessel and a second area value regarding a cross-section through which blood flows in the blood vessel;
   calculating, with respect to each of the plurality of cross-sections of the blood vessel, a ratio of the first area value to the second area value; and
   determining a cross-section having a smallest calculated ratio as a stenosis cross-section from among the plurality of cross-sections of the blood vessel.

6. The ultrasound imaging method of claim 1, wherein the first cross-section is spaced apart by a predetermined first interval in a first direction from the stenosis cross-section and the second cross-section is spaced apart by the first interval in a second direction that is opposite to the first direction from the stenosis cross-section.

7. The ultrasound imaging method of claim 1, wherein the determining of the first cross-section and the second cross-section comprises:

   detecting from the 3D ultrasound volume data a branch point where the blood vessel diverges into two; and
   determining a cross-section spaced apart by a third interval in a first direction from the branch point as the first cross-section and determining a cross-section spaced apart by the third interval in a second direction that is opposite to the first direction from the branch point as the second cross-section.

8.  An ultrasound imaging apparatus displaying an ultrasound image with respect to an object comprising a blood vessel, the ultrasound imaging apparatus comprising:

    an ultrasound data acquisition unit configured to obtain 3D ultrasound volume data with respect to the object;
    a processor configured to analyze a degree of stenosis for a plurality of cross-sections of the blood vessel from the 3D ultrasound volume data, determine a stenosis cross-section with a highest degree of stenosis and determine a first cross-section and a second cross-section that are spaced apart from each other in different directions from the stenosis cross-section from among the plurality of cross-sections of the blood vessel; and
    a display unit configured to display at least two cross-sections of the stenosis cross-section, the first cross-section, the second cross-section, and at least two stenosis index values showing a degree of stenosis of the blood vessel.

9.  The ultrasound imaging apparatus of claim 8, wherein the processor is further configured to calculate at least two index values from among a first index value representing a ratio of a diameter of the first cross-section to a difference between the diameter of the first cross-section and a diameter of the stenosis cross-section, a second index value representing a ratio of an entire blood vessel diameter defined by a blood vessel wall of the stenosis cross-section to a difference between the entire blood vessel diameter and the diameter of the stenosis cross-section, a third index value representing a ratio of a diameter of the second cross-section to a difference between the diameter of the second cross-section and the diameter of the stenosis cross-section, and a fourth index value representing a ratio of a cross-sectional area of the entire blood vessel of the stenosis cross-section to an area of the stenosis cross-section, and the display unit is further configured to display at least two index values from among the first index value, the second index value, the third index value, and the fourth index value.

10. The ultrasound imaging apparatus of claim 8, wherein the display unit is further configured to display a user interface that displays at least one of different colors, graphs, and figures based on sizes of the at least two stenosis index values, along with the at least two stenosis index values.

11. The ultrasound imaging apparatus of claim 8, wherein the ultrasound data acquisition unit is further configured to transmit ultrasound signals to the object, receive ultrasound echo signals reflected by the object, and obtain the 3D ultrasound volume data from the received ultrasound echo signals.

12. The ultrasound imaging apparatus of claim 8, wherein the processor is further configured to obtain the plurality of cross-sections of the blood vessel by segmenting the 3D ultrasound volume data at predetermined intervals, calculate, with respect to each of the plurality of cross-sections of the blood vessel, a first area value regarding a cross-section defined by a blood vessel wall of the blood vessel and a second area value regarding a cross-section through which blood flows in the blood vessel, calculate, with respect to each of the plurality of cross-sections of the blood vessel, a ratio of the first area value to the second area value, and determine a cross-section having a smallest calculated ratio as a stenosis cross-section from among the plurality of cross-sections of the blood vessel.

13. The ultrasound imaging apparatus of claim 8, wherein the first cross-section is spaced apart by a predetermined first interval in a first direction from the stenosis cross-section and the second cross-section is spaced apart by the first interval in a second direction that is opposite to the first direction from the stenosis cross-section.

14. The ultrasound imaging apparatus of claim 8, wherein the processor is further configured to detect from the 3D ultrasound volume data a branch point where the blood vessel diverges into two, determines a cross-section spaced apart by a third interval in a first direction from the branch point as the first cross-section, and determine a cross-section spaced apart by the third interval in a second direction that is opposite to the first direction from the branch point as the second cross-section.

15. A non-transitory computer-readable recording medium having recorded thereon a program for executing the method of claim 1 on a computer.

# FIG. 1

$P_{230}$
$P_{220}$
$P_{210}$

200A

230

$D_{240}$

$P_{230}$
$P_{210}$
$P_{210}$
$P_{220}$
$P_{240}$

| NASCET : 60 % | ECST : 40 % |
| CC : 80 % | AREA : 75 % |

$I_1$    $I_3$  $I_2$  $I_4$

130

130

1000

112

10

200

Y
X

FIG. 2

100

ULTRASOUND IMAGING APPARATUS

110

ULTRASOUND DATA
ACQUISITION UNIT

112

ULTRASOUND PROBE

114

DATA GENERATOR

120

PROCESSOR

130

DISPLAY UNIT

# FIG. 3

```
                    ( START )
                        |
                        v
  +--------------------------------------------------+
  |     OBTAIN 3D ULTRASOUND VOLUME DATA WITH        |—— S310
  |     RESPECT TO OBJECT INCLUDING BLOOD VESSEL     |
  +--------------------------------------------------+
                        |
                        v
  +--------------------------------------------------+
  |     ANALYZE DEGREE OF STENOSIS FOR PLURALITY OF  |
  |     CROSS-SECTIONS OF BLOOD VESSEL FROM 3D       |—— S320
  |     ULTRASOUND VOLUME DATA AND DETERMINE STENOSIS|
  |     CROSS-SECTION WITH HIGHEST DEGREE OF STENOSIS|
  +--------------------------------------------------+
                        |
                        v
  +--------------------------------------------------+
  | DETERMINE FIRST CROSS-SECTION AND SECOND CROSS-SECTION |
  | THAT ARE SPACED APART FROM EACH OTHER IN DIFFERENT     |—— S330
  | DIRECTIONS FROM STENOSIS CROSS-SECTION FROM            |
  | AMONG PLURALITY OF CROSS-SECTIONS OF BLOOD VESSEL      |
  +--------------------------------------------------+
                        |
                        v
  +--------------------------------------------------+
  | DISPLAY AT LEAST TWO CROSS-SECTIONS OF STENOSIS  |
  | CROSS-SECTION, FIRST CROSS-SECTION, AND          |
  | SECOND CROSS-SECTION AND AT LEAST TWO STENOSIS    |—— S340
  | INDEX VALUES REPRESENTING DEGREE OF STENOSIS OF BLOOD |
  | VESSEL ON DISPLAY UNIT OF ULTRASOUND IMAGING APPARATUS |
  +--------------------------------------------------+
                        |
                        v
                    ( END )
```

FIG. 4A

FIG. 4B

EP 3 199 108 A1

# FIG. 4C

# FIG. 5

```
                        ( START )
                            │
                            ▼
┌─────────────────────────────────────────────────────┐
│  OBTAIN PLURALITY OF CROSS-SECTIONS OF BLOOD         │
│  VESSEL BY DIVIDING 3D ULTRASOUND VOLUME DATA        │── S510
│         AT PREDETERMINED INTERVALS                   │
└─────────────────────────────────────────────────────┘
                            │
                            ▼
┌─────────────────────────────────────────────────────┐
│  CALCULATE, WITH RESPECT TO EACH OF PLURALITY OF     │
│  CROSS-SECTIONS OF BLOOD VESSEL, FIRST AREA VALUE    │
│  REGARDING CROSS-SECTION OF BLOOD VESSEL DEFINED     │── S520
│  BY BLOOD VESSEL WALL AND SECOND AREA VALUE          │
│  REGARDING INTERNAL REGION THROUGH WHICH BLOOD       │
│  FLOWS IN BLOOD VESSEL                               │
└─────────────────────────────────────────────────────┘
                            │
                            ▼
┌─────────────────────────────────────────────────────┐
│  CALCULATE RATIO OF FIRST AREA VALUE TO SECOND       │
│  AREA VALUE FOR EACH OF PLURALITY OF CROSS-          │── S530
│  SECTIONS OF BLOOD VESSEL                            │
└─────────────────────────────────────────────────────┘
                            │
                            ▼
┌─────────────────────────────────────────────────────┐
│  DETERMINE CROSS-SECTION HAVING SMALLEST             │
│  CALCULATED RATIO AS STENOSIS CROSS-SECTION FROM     │── S540
│  AMONG PLURALITY OF CROSS-SECTIONS OF BLOOD VESSEL   │
└─────────────────────────────────────────────────────┘
                            │
                            ▼
                        (  END  )
```

FIG. 6

FIG. 7

100

ULTRASOUND IMAGING APPARATUS

150

MEMORY

110

ULTRASOUND DATA ACQUISITION UNIT

120

PROCESSOR

130

DISPLAY UNIT

140

USER INPUT UNIT

# FIG. 8

```
                    ┌─────────┐
                    │  START  │
                    └────┬────┘
                         │
                         ▼
┌─────────────────────────────────────────────────┐
│ ANALYZE DEGREE OF STENOSIS FOR PLURALITY OF CROSS-│
│  SECTIONS OF BLOOD VESSEL FROM 3D ULTRASOUND      │── S810
│      VOLUME DATA AND DETERMINE STENOSIS           │
│   CROSS-SECTION WITH HIGHEST DEGREE OF STENOSIS   │
└─────────────────────┬───────────────────────────┘
                      │
                      ▼
┌─────────────────────────────────────────────────┐
│  RECEIVE USER INPUT FOR SETTING FIRST INTERVAL    │── S820
│   SPACED APART FROM STENOSIS CROSS-SECTION        │
└─────────────────────┬───────────────────────────┘
                      │
                      ▼
┌─────────────────────────────────────────────────┐
│  DETERMINE FIRST CROSS-SECTION SPACED APART       │
│  BY FIRST INTERVAL IN FIRST DIRECTION FROM STENOSIS│── S830
│  CROSS-SECTION AND SECOND CROSS-SECTION SPACED    │
│  APART BY FIRST INTERVAL IN SECOND DIRECTION FROM │
│ STENOSIS CROSS-SECTION, IN 3D ULTRASOUND VOLUME DATA│
└─────────────────────┬───────────────────────────┘
                      │
                      ▼
                 ┌─────────┐
                 │   END   │
                 └─────────┘
```

FIG. 9

# FIG. 10

```
                    ( START )
                        |
                        v
+-----------------------------------------------+
| OBTAIN PLURALITY OF CROSS-SECTIONS OF BLOOD   |---S1010
| VESSEL BY PROCESSING 3D ULTRASOUND VOLUME DATA|
+-----------------------------------------------+
                        |
                        v
+-----------------------------------------------+
| DETECT FROM 3D ULTRASOUND VOLUME DATA         |---S1020
| BRANCH POINT WHERE BLOOD VESSEL DIVERGES INTO |
| TWO                                           |
+-----------------------------------------------+
                        |
                        v
+-----------------------------------------------+
| DETERMINE CROSS-SECTION SPACED APART BY SECOND|
| INTERVAL IN FIRST DIRECTION FROM BRANCH POINT |
| AS FIRST CROSS-SECTION AND CROSS-SECTION      |---S1030
| SPACED APART BY SECOND INTERVAL IN SECOND     |
| DIRECTION OPPOSITE TO FIRST DIRECTION FROM    |
| BRANCH POINT AS SECOND CROSS-SECTION          |
+-----------------------------------------------+
                        |
                        v
                    ( END )
```

# FIG. 11A

FIG. 11B

# FIG. 12A

200A

130

131

132

$P_{230}$

$P_{230}$

$P_{220}$

$P_{210}$

$P_{210}$

$P_{220}$

$I_1$   NASCET : 60 %

$I_4$   AREA : 76 %

133

# FIG. 12B

## FIG. 12C

## FIG. 12D

FIG. 13

# FIG. 14

2000

**WIRELESS PROBE**

2100

**TRANSMITTER**

| 2110 | 2120 | 2130 |
|------|------|------|
| PULSER | TRANSMISSION DELAYING UNIT | PULSE GENERATOR |

10

2200

**TRANSDUCERS**

2300

**RECEIVER**

| 2310 | 2320 | 2330 | 2340 |
|------|------|------|------|
| AMPLIFIER | ADC | RECEPTION DELAYING UNIT | SUMMING UNIT |

Europäisches
Patentamt

European
Patent Office

Office européen
des brevets

# EUROPEAN SEARCH REPORT

Application Number

EP 16 18 7183

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 2 886 058 A1 (SAMSUNG MEDISON CO LTD [KR]) 24 June 2015 (2015-06-24) * the whole document * | 1-15 | INV. A61B8/08 |
| Y | US 6 503 202 B1 (HOSSACK JOHN A [US] ET AL) 7 January 2003 (2003-01-07) * the whole document * | 1-15 | |
| Y | IVAN N. STAIKOV ET AL: "Comparison of the ECST, CC, and NASCET grading methods and ultrasound for assessing carotid stenosis", J NEUROL, vol. 247, no. 9, 1 January 2000 (2000-01-01), pages 681-686, XP055378528, * the whole document * | 1-15 | |
| A | BARRATT DEAN C ET AL: "Reconstruction and Quantification of the Carotid Artery Bifurcation From 3-D Ultrasound Images", IEEE TRANSACTIONS ON MEDICAL IMAGING, vol. 23, no. 5, 1 May 2004 (2004-05-01), pages 567-583, XP011112233, ISSN: 0278-0062, DOI: 10.1109/TMI.2004.825601 * the whole document * | 4,5,7, 11,12,14 | |

TECHNICAL FIELDS
SEARCHED (IPC)

A61B

-/--

The present search report has been drawn up for all claims

| Place of search | Date of completion of the search | Examiner |
|---|---|---|
| Munich | 2 June 2017 | Küster, Gunilla |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 1 of 2

Europäisches
Patentamt

European
Patent Office

Office européen
des brevets

# EUROPEAN SEARCH REPORT

Application Number

EP 16 18 7183

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LOIZOU CHRISTOS ET AL: "An integrated system for the segmentation of atherosclerotic carotid plaque ultrasound video", IEEE TRANSACTIONS ON ULTRASONICS, FERROELECTRICS AND FREQUENCY CONTROL, vol. 61, no. 1, 1 January 2014 (2014-01-01), pages 86-101, XP011536256, ISSN: 0885-3010, DOI: 10.1109/TUFFC.2014.6689778 [retrieved on 2013-12-19] * the whole document * | 5,12 | |
| A | US 2014/324475 A1 (OCHI MASUMI [JP] ET AL) 30 October 2014 (2014-10-30) * paragraphs [0021] - [0028], [0065] - [0069]; figure 10 * | 1,8 | |

TECHNICAL FIELDS
SEARCHED (IPC)

The present search report has been drawn up for all claims

| Place of search | Date of completion of the search | Examiner |
|---|---|---|
| Munich | 2 June 2017 | Küster, Gunilla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 16 18 7183

This annex lists the patent family members relating to the patent documents cited in the above-mentioned European search report.
The members are as contained in the European Patent Office EDP file on
The European Patent Office is in no way liable for these particulars which are merely given for the purpose of information.

02-06-2017

| Patent document cited in search report | | Publication date | Patent family member(s) | | Publication date |
|---|---|---|---|---|---|
| EP 2886058 | A1 | 24-06-2015 | EP 2886058 A1<br>KR 20150071531 A<br>US 2015173716 A1 | | 24-06-2015<br>26-06-2015<br>25-06-2015 |
| US 6503202 | B1 | 07-01-2003 | NONE | | |
| US 2014324475 | A1 | 30-10-2014 | CN 103796595 A<br>JP 2014061289 A<br>US 2014324475 A1<br>WO 2014034911 A1 | | 14-05-2014<br>10-04-2014<br>30-10-2014<br>06-03-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020160012456